(19) Europäisches Patentamt
European Patent Office
Office européen des brevets

(11) EP 1 572 996 B1

(12) # EUROPEAN PATENT SPECIFICATION

(45) Date of publication and mention of the grant of the patent:
**11.06.2014 Bulletin 2014/24**

(21) Application number: **03812567.0**

(22) Date of filing: **10.12.2003**

(51) Int Cl.:
*C12N 9/42* (2006.01) *C11D 3/386* (2006.01)

(86) International application number:
**PCT/DK2003/000844**

(87) International publication number:
**WO 2004/053039 (24.06.2004 Gazette 2004/26)**

(54) **DETERGENT COMPOSITION COMPRISING ENDO-GLUCANASE**

DETERGENZ ZUSAMMENSETZUNG MIT ENDO-GLUCANASE

COMPOSITION DETERGENTE COMPRENANT UNE ENDO-GLUCANASE

(84) Designated Contracting States:
**AT BE BG CH CY CZ DE DK EE ES FI FR GB GR HU IE IT LI LU MC NL PT RO SE SI SK TR**

(30) Priority: **11.12.2002 DK 200201898**

(43) Date of publication of application:
**14.09.2005 Bulletin 2005/37**

(83) **Declaration under Rule 32(1) EPC (expert solution)**

(60) Divisional application:
**10177589.8 / 2 311 941**

(73) Proprietor: **Novozymes A/S**
**2880 Bagsvaerd (DK)**

(72) Inventors:
- **GIBSON, Keith**
  **2880 Bagsvaerd (DK)**
- **HANSEN, Lone**
  **2630 Tastrup (DK)**

(74) Representative: **Rasmussen, Preben et al**
**Novozymes A/S**
**Patents**
**Krogshoejvej 36**
**2880 Bagsvaerd (DK)**

(56) References cited:
**EP-A- 0 544 944** **EP-A- 0 921 188**
**EP-A1- 0 843 041** **WO-A-96/34080**
**WO-A1-96/29397** **WO-A1-98/12307**

- **DATABASE GENBANK [Online] 24 February 2003 (2003-02-24) Database accession no. CAD 67813 XP002276684 -& WO 02/099091 A (NOVOZYMES A/S) 12 December 2002 (2002-12-12)**
- **DATABASE GENBANK [Online] 18 December 2003 (2003-12-18) Database accession no. CAF 02232 XP002276685 -& EP 1 350 843 A (KAO CORP.) 8 October 2003 (2003-10-08)**
- **DATABASE GENBANK [Online] 19 December 2000 (2000-12-19) Database accession no. BAB 19360 XP002276686 -& HAKAMADA Y ET AL : "Deduced amino acid sequence and possible catalytic residues of a thermostable, alkaline cellulase from an alkaliphilic bacillus strain." BIOSCIENCE BIOTECHNOLOGY BIOCHEMISTRY, vol. 64, no. 11, November 2000 (2000-11), pages 2281-2289, XP002225730 JAPAN SOC. FOR BIOSCIENCE, BIOTECHNOLOGY AND AGROCHEM. TOKYO, JP. ISSN: 0916-8451**
- **DATABASE GENBANK [Online] 14 September 2001 (2001-09-14) Database accession no. AAA 73189 XP002276687 -& SUMITOMO N ET AL: "Nucleotide sequence of the gene for an alkaline endoglucanase from an alkalophilic bacillus and its expression in Escherichia coli and Bacillus subtilis." BIOSCIENCE BIOTECHNOLOGY BIOCHEMISTRY, vol. 56, no. 6, June 1992 (1992-06), pages 872-877, XP002225731 JAPAN SOC. FOR BIOSCIENCE, BIOTECHNOLOGY AND AGROCHEM. TOKYO, JP. ISSN: 0916-8451**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE WPI Section Ch, Week 200057 Derwent Publications Ltd., London, GB; Class D16, AN 2000-596504 XP002276688 & JP 2000 210081 A (KAO CORP), 2 August 2000 (2000-08-02)**

## Description

The present invention relates to a detergent composition comprising an endo-glucanase that provides improved detergency performance. The invention also relates to a detergent composition comprising a combination of an endo-glucanase and an alpha- amylase. One aspect of the invention relates to a process of washing a fabric or hard surface with the endo-glucanase containing detergent.

## BACKGROUND OF THE INVENTION

An important objective of a washing process is to transfer, as completely as possible, the soil from the object being washed into the washing solution, such that the soil can then be discarded with the wash solution. A wash process which merely redistributes the soil on the object being washed is obviously not satisfactory. Thus evaluations of detergency performance, i.e. the performance of washing processes, must include consideration both of the removal of soil from the object being washed and of the redeposition of this soil onto the object being washed or onto the equipment being used for the wash process.

With some soils and some surfaces it is very difficult to achieve a satisfactory detergency performance. Problem soils include particulate soils such as clays and carbon. Visible soils are often difficult to wash away completely because they are bound to, or attracted to, a surface by traces of non-visible, sticky substances. Problem surfaces include the surfaces of cotton fibres.

To help overcome such problem soils, detergent formulations may include an anti-redeposition agent. The anti-redeposition agent is added in order to ensure that the above mentioned problem soils, once detached from the fabrics, can be kept dissolved or suspended in the wash liquor in such a way that they are not re-deposited on the cleaned fabric.

To obtain both an anti-redeposition effect and a cleaning effect it has been suggested to add a mixture of cellulases to the detergent, one cellulase having anti-redeposition effect and one having a cellulose-degrading effect. EP 822 973 relates to a detergent composition comprising a mixture of cellulases and optionally also containing additional enzymes. However, there is a need for improved combinations of enzymes having anti-redeposition effect and other enzymes, such as proteases, amylases, hemi-cellulases, lipases and pectinases.

Enzymes are commonly used to remove the sticky substances that increase soil binding. There is a need for improved enzyme performance in terms of soil removal and/or prevention of soil redeposition.

International publication number WO 02/09909 concerns an enzyme exhibiting endo-beta-1,4-glucanase activity.

European patent application published under number EP 1350843 discloses alkaline cellulose variants.

## SUMMARY OF THE INVENTION

The invention relates to a detergent composition comprising an anti-redeposition endo-glucanase, i.e. an endo-glucanase having anti-redeposition effect. The inventors have found that such endo-glucanase containing detergents give advantages when used for washing fabrics (especially laundry washing) or hard surfaces (especially automatic dish washing).

In the context of the present invention, an endo-glucanase having anti-redeposition effect is characterised by its ability to prevent redeposition in a wash test.

The inventors also have found that detergent compositions comprising specific combinations of certain endo-glucanase(s) having anti-redeposition effect and certain cellulase(s) having increased stability towards anionic tensides such as linear (straight-chain) alkyl benzene sulfonates (often referred to as "LAS"), have advantages compared to the prior art detergent compositions described in EP 822 973. For instance, a decreased amount of enzyme protein is necessary to obtain the desired cleaning and anti-redeposition effect. This results in improved product economy.

The inventors also have found that it is surprisingly advantageous to include an endo-glucanase having anti-redeposition effect in detergents that also contain other enzymes. These other enzymes include, for example, enzymes that are classified as protease, amylase, beta-glucanase, lipase, hemi-cellulase, cutinase, pectinase and pectate lyase.

Thus in a first aspect the invention relates to the use of a detergent composition comprising an anti-redeposition endo-glucanase having the amino acid sequence of SEQ ID NO: 2, or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1 and an alpha-amylase to provide improved detergency performance on stains that contain starch, said use being characterized in that the detergency performance obtained from the combination of an anti-redeposition endo-glucanase and an alpha-amylase is higher than the detergency performance of the same detergent composition without the anti-redeposition endo-glucanase and said detergency performance is determined by the wash test method of Example 7.

In a second aspect the invention relates to the use of detergent composition containing both an endo-glucanase as defined above and an alpha-amylase and optionally one or more other enzyme from classes such as, but not limited to protease, amylase, beta-glucanase, lipase, hemi-cellulase, cutinase, pectinase and pectate lyase.

In an aspect the invention relates to process for washing a fabric, comprising contacting a fabric with an aqueous solution of a detergent composition comprising an anti-redeposition endo-glucanase having the amino acid sequence of SEQ ID NO: 2; or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1 and an alpha-amylase, for an effective period of time.In an aspect the invention relates to a process for washing a hard surface, comprising contacting the surface with an aqueous solution of a detergent composition comprising an anti-redeposition endo-glucanase having the amino acid sequence of SEQ ID NO: 2; or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1 and an alpha-amylasefor an effective period of time.

## DETAILED DESCRIPTION OF THE INVENTION

The invention relates to a detergent composition comprising an endo-glucanase having anti-redeposition effect.

<u>The endo-glucanase with anti-redeposition effect</u>

The endo-glucanase having anti-redeposition effect is according to the invention selected from one of:

amino acid sequence of SEQ ID NO: 2, or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1

In a preferred embodiment the endo-glucanase is derived from *Bacillus* sp. AA349, DSM 12648 and also shown in SEQ ID NO: 2 herein or a sequence being 90% identical thereto.

The strain *Bacillus* sp. AA349, which has been isolated from a soil sample originating in Greece, was deposited by the inventors according to the Budapest Treaty on the International Recognition of the Deposit of Microorganisms for the Purposes of Patent Procedure at the Deutsche Sammlung von Mikroorganismen und Zellkulturen GmbH, Mascheroder Weg 1b, D-38124 Braunschweig, Germany, on 25 January 1999 under the deposition number DSM 12648.

The deposit was made by Novo Nordisk A/S and was later assigned to Novozymes A/S.

<u>Detergent compositions comprising anionic tensides</u>

A preferred embodiment the invention relates to the use of a detergent composition comprising anionic tensides, and to a combination of an endo-glucanase as defined above and a fungal cellulase, wherein both enzymes are stable in the presence of anionic tensides, such as LAS.

For the purpose of the present invention, enzymes that are stable in LAS are defined by a LAS stability test. Enzymes that give a LAS stability result of at least 50% are regarded as being LAS stable.

Detergents commonly contain more than one type of surfactant, for example combinations of both anionic and nonionic surfactants. In a preferred embodiment the invention relates to detergent formulations in which the ratio (based on weight) between anionic and nonionic surfactants is >1:1, preferably >1.5:1.

Thus, in a preferred embodiment the invention relates to a detergent composition wherein

(a) the endo-glucanase is selected from one of:

(i) the endo-glucanase having the amino acid sequence of position 1 to position 773 of SEQ ID NO: 2;
(ii) an endo-glucanase having a sequence of at least 90% identity to the amino acid sequence of position 1 to position 773 of SEQ ID NO:2; or a fragment thereof that has endo-glucanase activity; and

(b) the cellulase is selected from one of:

(i) the cellulase having the amino acid sequence of position 1 to position 299 of SEQ ID NO: 4; or

(ii) a cellulase having a sequence of at least 70% identity to the amino acid sequence of position 1 to position 299 of SEQ ID NO:4, or a fragment thereof that has cellulase activity.

Alternatively, the endo-glucanase of (a)(i) above is derived from *Bacillus* sp. KSMS237 deposited as FERM P-16067 (with the Patent Microorganism Depository, National Institute of Bioscience and Human-Technology, Agency of Industrial Science & Industry, Tsukuba-shi, Ibaraki, 305 Japan) and shown in position 1 to 824 of SEQ ID NO: 1 of JP 2000210081 A.

Other alternative cellulases are the LAS resistant variants disclosed in Example 8 of WO 98/12307. Preferred embodiments of the invention comprise these alternative cellulases in combination with the endo-glucanases of (a) above.

In a preferred embodiment the cellulase is a *Thielavia terrestris* cellulase, preferably the cellulase disclosed in SEQ ID NO: 9 in WO 96/29397 and SEQ ID NO: 4 herein or an enzyme with at least 70% identity thereto. In a preferred embodiment the cellulase is the *Thielavia terrestris* variant disclosed in Example 1 of WO 98/12307.

Identity

In the context of the present invention the degree of identity is determined between two sequences indicating a derivation of the first sequence from the second. The identity is determined by means of the computer program GAP provided in the GCG program package. Thus, Gap GCGv8 is used with the following default parameters: GAP creation penalty of 3.0 and GAP extension penalty of 0.1, the default scoring matrix, for protein sequences. GAP uses the method of Needleman/Wunsch/Sellers to make alignments.

Detergent compositions comprising other enzymes

Various types of enzymes are very commonly included in detergent formulations for laundry cleaning and for hard surface cleaning. One purpose of these enzymes is to degrade soil binding substances. These substances bind or attract soil and stains onto the fabric or hard surface. The degraded substances, and the associated soil and stain, are then more easily removed during the washing process. Redeposition of soil, which can be increased by soil binding substances, is prevented.

The substance on which an enzyme acts is called the substrate. Enzymes are known to be "substrate specific", i.e. each class of enzyme can only degrade one class of substances. For example, a protease can degrade proteins but cannot degrade starch. An amylase can degrade starch but cannot degrade proteins.

Because the soils and stains that are important for detergent formulators can contain many kinds of soil binding substances, a range of different enzymes, all with different substrate specificities have been developed for use in detergents. These include enzymes such as protease, amylase, beta-glucanase, lipase, hemi-cellulase, cutinase, pectinase and pectate lyase.

Surprisingly it has been found that the endo-glucanase of the invention are not substrate specific, in that they can provide detergency benefits on a wide range of soils and stains when used in combination with other enzymes.

The endo-glucanase of the present invention includes, in addition to the enzymes specified by reference to SEQ ID NO 2, other endo-glucanases with surprisingly high anti-redeposition effect. Two tests are provided in order to describe and identify these enzymes: 1) a test for endo-glucanase activity, and 2) a test for anti-redeposition effect. Enzymes which provide greater than the specified minimum performance on both these tests are regarded, for the purpose of this invention, as endo-glucanases having anti-redeposition effect.

Thus in a preferred embodiment an endo-glucanase of the invention is used together with an amylase to provide improved detergency performance on soils that contain starch. Such amylases comprise e.g. $\alpha$- or $\beta$-amylases of bacterial or fungal origin. Chemically or genetically modified mutants of such amylases are included in this connection. Relevant $\alpha$-amylases include, for example, $\alpha$-amylases obtainable from *Bacillus* species, in particular a special strain of B. *licheniformis,* described in more detail in GB 1296839. Relevant commercially available amylases include Natalase™, Stainzyme®, Duramyl®, Termamyl®, Termamyl™ Ultra, Fungamyl® and BAN® (all available from Novozymes A/S, Bagsvaerd, Denmark), and Rapidase™ and Maxamyl™ P (available from DSM, Holland).

In a preferred embodiment the alpha-amylase is derived from *Bacillus* sp. strains NCIB 12289, NCIB 12512, NCIB 12513 and DSM 9375. Especially preferred are the alpha-amylases shown in SEQ ID NOS 1 and 2 of WO 95/26397.

In another preferred embodiment the alpha-amylase is the AA560 alpha-amylase derived from *Bacillus* sp. DSM 12649 disclosed as SEQ ID NO: 2 in WO 00/60060 . Especially preferred are variants of the AA560 alpha-amylase, including the AA560 variant disclosed in Example 7 and 8

Other useful amylases are CGTases (cyclodextrin glucanotransferases, EC 2.4.1.19), e.g. those obtainable from species of *Bacillus, Thermoanaerobactor* or *Thermoanaerobacterium.*

In another preferred embodiment an endo-glucanase of the invention is used together with a protease to provide improved detergency performance on soils that contain protein. Such proteases comprise those of animal, vegetable

or microbial origin. Proteases of microbial origin are preferred. Chemically or genetically modified mutants of such proteases are included in this connection. The protease may be a serine protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270.

Relevant commercially available protease enzymes include Primase®, Durazym®, Everlase®, Kannase®, Alcalase®, Savinase® and Esperase® (all available from Novozymes A/S, Bagsvaerd, Denmark), Maxatase™, Maxacal™, Maxapem™ and Properase™ (available from DSM, Holland), Purafect™ and Purafect™ OXP (available from Genencor International, USA), and Opticlean™ and Optimase™ (available from by Solvay Enzymes).

In another preferred embodiment an endo-glucanase of the invention is used together with a lipase to provide improved detergency performance on soils that contain fat or oil. Such lipases comprise those of bacterial or fungal origin. Chemically or genetically modified mutants of such lipases are included in this connection.

Examples of useful lipases include a *Humicola lanuginosa* lipase, e.g. as described in EP 258 068 and EP 305 216; a *Rhizomucor miehei* lipase, e.g. as described in EP 238 023; a *Candida* lipase, such as a C. *antarctica* lipase, e.g. the C. *antarctica* lipase A or B described in EP 214 761; a *Pseudomonas lipase,* such as one of those described in EP 721 981 (e.g. a lipase obtainable from a *Pseudomonas* sp. SD705 strain having deposit accession number FERM BP-4772), in PCT/JP96/00426, in PCT/JP96/00454 (e.g. a *P. solanacearum* lipase), in EP 571 982 or in WO 95/14783 (e.g. a *P. mendocina* lipase), a *P. alcaligenes* or *P. pseudoalcaligenes* lipase, e.g. as described in EP 218 272, a *P. cepacia* lipase, e.g. as described in EP 331 376, a *P. stutzeri* lipase, e.g. as disclosed in GB 1,372,034, or a *P. fluorescens* lipase; a *Bacillus* lipase, e.g. a *B. subtilis* lipase (Dartois et al. (1993) Biochemica et Biophysica Acta 1131:253-260), a *B. stearothermophilus* lipase (JP 64/744992) and a *B. pumilus* lipase (WO 91/16422).

Other potentially useful types of lipolytic enzymes include cutinases, e.g. a cutinase derived from *Pseudomonas mendocina* as described in WO 88/09367, or a cutinase derived from *Fusarium solani f. pisi* (described, e.g., in WO 90/09446).

Suitable commercially available lipases include Lipex®, Lipolase® and Lipolase Ultra® (available from Novozymes A/S), M1 Lipase™ and Lipomax™ (available from Genencor Inc.) and Lipase P "Amano" (available from Amano Pharmaceutical Co. Ltd.). Commercially available cutinases include Lumafast™ from Genencor Inc.

In another preferred embodiment an endo-glucanase of the invention is used together with a hemi-cellulase to provide improved detergency performance on soils that contain hemicellulose and similar polysaccharides. Such hemicellulases include xylanases, xyloglucanases, arabinofuranosidases, acetyl xylan esterases, glucuronidases, ferulic acid esterases, coumaric acid esterases, endo-galactanases, mannanases, endo- or exo-arabinanases, exo-galactanases. Suitable mannanases include those of bacterial or fungal origin. Chemically or genetically modified mutants are included.

In a preferred embodiment the mannanase is derived from a strain of the genus *Bacillus,* especially *Bacillus* sp. I633 disclosed in positions 31-330 of SEQ ID NO:2 or in SEQ ID NO: 5 of WO 99/64619 or *Bacillus agaradhaerens,* for example from the type strain DSM 8721. A suitable mannanase is Mannaway® produced by Novozymes A/S.

Thus in a preferred embodiment an endo-glucanase of the invention is used together with a beta-glucanase (EC 3.2.1.6) to provide improved detergency performance on soils that contain beta-glucans. Further preferred beta-glucanases include lichenases and laminarinases.

Thus in a preferred embodiment an endo-glucanase of the invention is used together with pectinolytic enzymes such as a protopectinase, pectinase, polygalacturonase or pectate lyase to provide improved detergency performance on pectinaceous soils. Suitable pectinolytic enzymes include those described in WO 99/27083, WO 99/27084, WO 00/55309 and WO 02/092741. Suitable pectate lyases include those of bacterial or fungal origin. Chemically or genetically modified mutants are included.

In a preferred embodiment the pectate lyase is derived from a strain of the genus *Bacillus,* especially a strain of *Bacillus substilis,* especially *Bacillus subtilis* DSM14218 disclosed in SEQ ID NO:2 or a variant thereof disclosed in Example 6 of WO 02/092741.

Additionally, in a preferred embodiment an endo-glucanase of the invention is used together with other enzymes of the classes: pectate lyase (EC 4.2.2.2), pectin lyase (EC 4.2.2.10), rhamnogalacturonan lyase (EC not defined), endo-1,4-galactanase (EC 3.2.1.89), xyloglucanase (EC not defined), xylanase (EC 3.2.1.8), arabinanase (EC 3.2.1.99), alpha-L-arabinofuranosidase (EC 3.2.1.55), Mannan endo-1,4-mannosidase (EC 3.2.1.78), beta-mannosidase (EC 3.2.1.25), beta-1,3-1,4-glucanase (EC 3.2.1.73), rhamnogalacturonan hydrolase, exo-polygalacturonase (EC 3.2.1.67), rhamnogalacturonase (EC not defined), Glucan 1,3-beta-glucosidase (EC 3.2.1.58), Glucan endo-1,6-beta-glucosidase (EC 3.2.1.75), Mannan endo-1,4-beta-mannosidase (EC 3.2.1.78), Endo-1,4-beta-xylanase (EC 3.2.1.8), Cellulose 1,4-cellobiosidase (EC 3.2.1.91), cellobiohydrolase (EC 3.2.1.91). Polygalacturonases (EC 3.2.1.15). Acetyl and methyl esterase enzymes such as: rhamnogalacturonan methyl esterase, rhamnogalacturonan acetyl esterase, pectin methylesterase (EC 3.1.1.11), pectin acetylesterase (EC not defined), xylan methyl esterase, acetyl xylan esterase (EC 3.1.1.72), feruloyl esterase (EC 3.1.1.73), cinnamoyl esterase (EC 3.1.1.73) to provide improved detergency performance

on corresponding soils.

Detergent composition of the invention

The detergent compositions according to the present invention comprise a surfactant system, wherein the surfactant can be selected from nonionic and/or anionic and/or cationic and/or amphoteric and/or zwitterionic and/or semi-polar surfactants.

The surfactant is typically present at a level from 0.1 % to 60% by weight.

The surfactant is preferably formulated to be compatible with enzyme components present in the composition. In liquid or gel compositions the surfactant is most preferably formulated in such a way that it promotes, or at least does not degrade, the stability of any enzyme in these compositions.

Preferred systems to be used according to the present invention comprise as a surfactant one or more of the nonionic and/or anionic surfactants described herein.

Polyethylene, polypropylene, and polybutylene oxide condensates of alkyl phenols are suitable for use as the nonionic surfactant of the surfactant systems of the present invention, with the polyethylene oxide condensates being preferred. These compounds include the condensation products of alkyl phenols having an alkyl group containing from about 6 to about 14 carbon atoms, preferably from about 8 to about 14 carbon atoms, in either a straight chain or branched-chain configuration with the alkylene oxide. In a preferred embodiment, the ethylene oxide is present in an amount equal to from about 2 to about 25 moles, more preferably from about 3 to about 15 moles, of ethylene oxide per mole of alkyl phenol. Commercially available nonionic surfactants of this type include Igepal™ CO-630, marketed by the GAF Corporation; and Triton™ X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with about 1 to about 25 moles of ethylene oxide are suitable for use as the nonionic surfactant of the nonionic surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol™ 15-S-9 (The condensation product of $C_{11}$-$C_{15}$ linear alcohol with 9 moles ethylene oxide), Tergitol™ 24-L-6 NMW (the condensation product of $C_{12}$-$C_{14}$ primary alcohol with 6 moles ethylene oxide with a narrow molecular weight distribution), both marketed by Union Carbide Corporation; Neodol™ 45-9 (the condensation product of $C_{14}$-$C_{15}$ linear alcohol with 9 moles of ethylene oxide), Neodol™ 23-3 (the condensation product of $C_{12}$-$C_{13}$ linear alcohol with 3.0 moles of ethylene oxide), Neodol™ 45-7 (the condensation product of $C_{14}$-$C_{15}$ linear alcohol with 7 moles of ethylene oxide), Neodol™ 45-5 (the condensation product of $C_{14}$-$C_{15}$ linear alcohol with 5 moles of ethylene oxide) marketed by Shell Chemical Company, Kyro™ EOB (the condensation product of $C_{13}$-$C_{15}$ alcohol with 9 moles ethylene oxide), marketed by The Procter & Gamble Company, and Genapol LA 050 (the condensation product of $C_{12}$-$C_{14}$ alcohol with 5 moles of ethylene oxide) marketed by Hoechst. Preferred range of HLB in these products is from 8-11 and most preferred from 8-10.

Also useful as the nonionic surfactant of the surfactant systems of the present invention are alkylpolysaccharides disclosed in US 4,565,647, having a hydrophobic group containing from about 6 to about 30 carbon atoms, preferably from about 10 to about 16 carbon atoms and a polysaccharide, e.g. a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units.

The preferred alkylpolyglycosides have the formula

$$R^2O(C_nH_{2n}O)_t(glycosyl)_x$$

wherein $R^2$ is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between

their 1-position and the preceding glycosyl units 2-, 3-, 4-, and/or 6-position, preferably predominantly the 2-position.

The condensation products of ethylene oxide with a hydrophobic base formed by the condensation of propylene oxide with propylene glycol are also suitable for use as the additional nonionic surfactant systems of the present invention. The hydrophobic portion of these compounds will preferably have a molecular weight from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially available Pluronic™ surfactants, marketed by BASF.

Also suitable for use as the nonionic surfactant of the nonionic surfactant system of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available Tetronic™ compounds, marketed by BASF.

Preferred for use as the nonionic surfactant of the surfactant systems of the present invention are polyethylene oxide condensates of alkyl phenols, condensation products of primary and secondary aliphatic alcohols with from about 1 to about 25 moles of ethyleneoxide, alkylpolysaccharides, and mixtures hereof. Most preferred are $C_8$-$C_{14}$ alkyl phenol ethoxylates having from 3 to 15 ethoxy groups and $C_8$-$C_{18}$ alcohol ethoxylates (preferably $C_{10}$ avg.) having from 2 to 10 ethoxy groups, and mixtures thereof.
Highly preferred nonionic surfactants are polyhydroxy fatty acid amide surfactants of the formula

$$R^2 - \underset{\displaystyle 0}{\overset{}{\underset{\|}{C}}} - \underset{\displaystyle R^1}{\underset{\|}{N}} - Z,$$

wherein $R^1$ is H, or $R^1$ is $C_{1-4}$ hydrocarbyl, 2-hydroxyethyl, 2-hydroxypropyl or a mixture thereof, $R^2$ is $C_{5-31}$ hydrocarbyl, and Z is a polyhydroxyhydrocarbyl having a linear hydrocarbyl chain with at least 3 hydroxyls directly connected to the chain, or an alkoxylated derivative thereof. Preferably, $R^1$ is methyl, $R^2$ is straight $C_{11-15}$ alkyl or $C_{16-18}$ alkyl or alkenyl chain such as coconut alkyl or mixtures thereof, and Z is derived from a reducing sugar such as glucose, fructose, maltose or lactose, in a reductive amination reaction.

Highly preferred anionic surfactants include alkyl alkoxylated sulfate surfactants. Examples hereof are water soluble salts or acids of the formula $RO(A)_mSO_3M$ wherein R is an unsubstituted $C_{10}$-C-$_{24}$ alkyl or hydroxyalkyl group having a $C_{10}$-$C_{24}$ alkyl component, preferably a $C_{12}$-$C_{20}$ alkyl or hydro-xyalkyl, more preferably $C_{12}$-$C_{18}$ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are $C_{12}$-$C_{18}$ alkyl polyethoxylate (1.0) sulfate ($C_{12}$-$C_{18}E(1.0)M$), $C_{12}$-$C_{18}$ alkyl polyethoxylate (2.25) sulfate ($C_{12}$-$C_{18}(2.25)M$, and $C_{12}$-$C_{18}$ alkyl polyethoxylate (3.0) sulfate ($C_{12}$-$C_{18}E(3.0)M$), and $C_{12}$-$C_{18}$ alkyl polyethoxylate (4.0) sulfate ($C_{12}$-$C_{18}E(4.0)M$), wherein M is conveniently selected from sodium and potassium.

Suitable anionic surfactants to be used are alkyl ester sulfonate surfactants including linear esters of $C_8$-$C_{20}$ carboxylic acids (i.e., fatty acids) which are sulfonated with gaseous $SO_3$ according to "The Journal of the American Oil Chemists Society", 52 (1975), pp. 323-329. Suitable starting materials would include natural fatty substances as derived from tallow, palm oil, etc.

The preferred alkyl ester sulfonate surfactant, especially for laundry applications, comprises alkyl ester sulfonate surfactants of the structural formula:

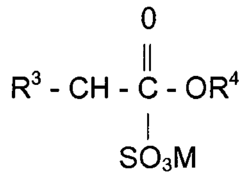

wherein $R^3$ is a $C_8$-$C_{20}$ hydrocarbyl, preferably an alkyl, or combination thereof, $R^4$ is a $C_1$-$C_6$ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethonolamine, and triethanolamine. Preferably, $R^3$ is $C_{10}$-$C_{16}$ alkyl, and $R^4$ is methyl, ethyl or isopropyl. Especially preferred are the methyl ester sulfonates wherein $R^3$ is $C_{10}$-$C_{16}$ alkyl.

Other suitable anionic surfactants include the alkyl sulfate surfactants which are water soluble salts or acids of the formula $ROSO_3M$ wherein R preferably is a $C_{10}$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of $C_{12}$-$C_{16}$ are preferred for lower wash temperatures (e.g. below about 50°C) and $C_{16}$-$C_{18}$ alkyl chains are preferred for higher wash temperatures (e.g. above about 50°C).

Other anionic surfactants useful for detersive purposes can also be included in the laundry detergent compositions of the present invention. Theses can include salts (including, for example, sodium, potassium, ammonium, and substituted ammonium salts such as mono-di- and triethanolamine salts) of soap, $C_8$-$C_{22}$ primary or secondary alkanesulfonates, $C_8$-$C_{24}$ olefinsulfonates, sulfonated polycarboxylic acids prepared by sulfonation of the pyrolyzed product of alkaline earth metal citrates, e.g., as described in British patent specification No. 1,082,179, $C_8$-$C_{24}$ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated $C_{12}$-$C_{18}$ monoesters) and diesters of sulfosuccinates (especially saturated and unsaturated $C_6$-$C_{12}$ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula $RO(CH_2CH_2O)_k$-$CH_2C00$-M)+ wherein R is a $C_8$-$C_{22}$ alkyl, k is an integer from 1 to 10, and M is a soluble salt forming cation. Resin acids and hydrogenated resin acids are also suitable, such as rosin, hydrogenated rosin, and resin acids and hydrogenated resin acids present in or derived from tall oil.

Alkylbenzene sulfonates are highly preferred. Especially preferred are linear (straight-chain) alkyl benzene sulfonates (LAS) wherein the alkyl group preferably contains from 10 to 18 carbon atoms.

Further examples are described in "Surface Active Agents and Detergents" (Vol. I and II by Schwartz, Perrry and Berch). A variety of such surfactants are also generally disclosed in US 3,929,678, (Column 23, line 58 through Column 29, line 23,).

When included therein, the detergent compositions of the present invention typically comprise from about 1% to about 40%, preferably from about 3% to about 25 % by weight of such anionic surfactants.

The laundry detergent compositions of the present invention may also contain cationic, amphoteric, zwitterionic, and semi-polar surfactants, as well as the nonionic and/or anionic surfactants other than those already described herein.

Cationic detersive surfactants suitable for use in the laundry detergent compositions of the present invention are those having one long-chain hydrocarbyl group. Examples of such cationic surfactants include the ammonium surfactants such as alkyltrimethylammonium halogenides, and those surfactants having the formula:

$$[R^2(OR^3)_y][R^4(OR^3)_y]_2R^5N^+X^-$$

wherein $R^2$ is an alkyl or alkyl benzyl group having from about 8 to about 18 carbon atoms in the alkyl chain, each $R^3$ is selected form the group consisting of $-CH_2CH_2-$, $-CH_2CH(CH_3)-$,$-CH_2CH(CH_2OH)-$, $-CH_2CH_2CH_2-$, and mixtures thereof; each $R^4$ is selected from the group consisting of $C_1$-$C_4$ alkyl, $C_1$-$C_4$ hydroxyalkyl, benzyl ring structures formed by joining the two $R^4$ groups, $-CH_2CHOHCHOHCOR^6CHOHCH_2OH$, wherein $R^6$ is any hexose or hexose polymer having a molecular weight less than about 1000, and hydrogen when y is not 0; $R^5$ is the same as $R^4$ or is an alkyl chain, wherein the total number of carbon atoms of $R^2$ plus $R^5$ is not more than about 18; each y is from 0 to about 10,and the sum of the y values is from 0 to about 15; and X is any compatible anion.

Highly preferred cationic surfactants are the water soluble quaternary ammonium compounds useful in the present composition having the formula:

$$R_1R_2R_3R_4N^+X^- \qquad (i)$$

wherein $R_1$ is $C_8$-$C_{16}$ alkyl, each of $R_2$, $R_3$ and $R_4$ is independently $C_1$-$C_4$ alkyl, $C_1$-$C_4$ hydroxy alkyl, benzyl, and -$(C_2H_{40})_x$H where x has a value from 2 to 5, and X is an anion. Not more than one of $R_2$, $R_3$ or $R_4$ should be benzyl.

The preferred alkyl chain length for $R_1$ is $C_{12}$-$C_{15}$, particularly where the alkyl group is a mixture of chain lengths derived from coconut or palm kernel fat or is derived synthetically by olefin build up or OXO alcohols synthesis.

Preferred groups for $R_2$, $R_3$ and $R_4$ are methyl and hydroxyethyl groups and the anion X may be selected from halide, methosulphate, acetate and phosphate ions.

Examples of suitable quaternary ammonium compounds of formulae (i) for use herein are:

coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
$C_{12-15}$ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)$_4$ ammonium chloride or bromide;
choline esters (compounds of formula (i) wherein $R_1$ is

$CH_2$-$CH_2$-O-C-$C_{12-14}$ alkyl and $R_2R_3R_4$ are methyl).
||
O

di-alkyl imidazolines [compounds of formula (i)].

Other cationic surfactants useful herein are also described in US 4,228,044 and in EP 000224.

When included therein, the detergent compositions of the present invention typically comprise from 0.2% to about 25%, preferably from about 1% to about 8% by weight of such cationic surfactants.

Amphoteric surfactants are also suitable for use in the detergent compositions of the present invention. These surfactants can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about 8 to about 18 carbon atoms, and at least one contains an anionic water-solubilizing group, e.g. carboxy, sulfonate, sulfate. See US 3,929,678 (column 19, lines 18-35) for examples of amphoteric surfactants.

When included therein, the detergent compositions of the present invention typically comprise from 0.2% to about 15%, preferably from about 1% to about 10% by weight of such amphoteric surfactants.

Zwitterionic surfactants are also suitable for use in detergent compositions. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See US 3,929,678 (column 19, line 38 through column 22, line 48) for examples of zwitterionic surfactants.

When included therein, the detergent compositions of the present invention typically comprise from 0.2% to about 15%, preferably from about 1% to about 10% by weight of such zwitterionic surfactants.

Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms; water soluble phosphine oxides containing one alkyl moiety of from about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety from about 10 to about 18 carbon atoms and a moiety selected from the group consisting of alkyl and hydroxyalkyl moieties of from about 1 to about 3 carbon atoms.

Semi-polar nonionic detergent surfactants include the amine oxide surfactants having the formula:

$$R^3(OR^4)xN(R^5)2 \quad (\text{with } \overset{O}{\uparrow} \text{ on N})$$

wherein $R^3$ is an alkyl, hydroxyalkyl, or alkyl phenyl group or mixtures thereof containing from about 8 to about 22 carbon atoms; $R^4$ is an alkylene or hydroxyalkylene group containing from about 2 to about 3 carbon atoms or mixtures thereof; x is from 0 to about 3: and each $R^5$ is an alkyl or hydroxyalkyl group containing from about 1 to about 3 carbon atoms or a polyethylene oxide group containing from about 1 to about 3 ethylene oxide groups. The $R^5$ groups can be attached to each other, e.g., through an oxygen or nitrogen atom, to form a ring structure.

These amine oxide surfactants in particular include $C_{10}$-$C_{18}$ alkyl dimethyl amine oxides and $C_8$-$C_{12}$ alkoxy ethyl dihydroxy ethyl amine oxides.

When included therein, the detergent compositions of the present invention typically comprise from 0.2% to about 15%, preferably from about 1% to about 10% by weight of such semi-polar nonionic surfactants.

<u>Builder system</u>

The compositions according to the present invention may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethylenephosphonic acid. Though less preferred for obvious environmental reasons, phosphate builders can also be used herein.

Suitable builders can be an inorganic ion exchange material, commonly an inorganic hydrated aluminosilicate material, more particularly a hydrated synthetic zeolite such as hydrated zeolite A, X, B, HS or MAP.

Another suitable inorganic builder material is layered silicate, e.g. SKS-6 (Hoechst). SKS-6 is a crystalline layered silicate consisting of sodium silicate ($Na_2Si_2O_5$).

Suitable polycarboxylates containing one carboxy group include lactic acid, glycolic acid and ether derivatives thereof as disclosed in Belgian Patent Nos. 831, 368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in DE 2,446,686, and 2,446,487, US 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.

Polycarboxylates containing four carboxy groups include oxydisuccinates disclosed in British Patent No. 1,261,829, 1,1,2,2,-ethane tetracarboxylates, 1,1,3,3-propane tetracarboxylates containing sulfo substituents include the sulfosuccinate derivatives disclosed in British Patent Nos. 1,398,421 and 1,398,422 and in US 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis-cis-tetracarboxylates, cyclopentadienide pentacarboxylates, 2,3,4,5-tetrahydro-furan - cis, cis, cis-tetracarboxylates, 2,5-tetrahydro-furan-cis, discarboxylates, 2,2,5,5,-tetrahydrofuran - tetracarboxylates, 1,2,3,4,5,6-hexane - hexacarboxylates and carboxymethyl derivatives of polyhydric alcohols such as sorbitol, mannitol and xylitol. Aromatic polycarboxylates include mellitic acid, pyromellitic acid and the phthalic acid derivatives disclosed in British Patent No. 1,425,343.

Of the above, the preferred polycarboxylates are hydroxy-carboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

A suitable chelant for inclusion in the detergent compositions in accordance with the invention is ethylenediamine-N,N'-disuccinic acid (EDDS) or the alkali metal, alkaline earth metal, ammonium, or substituted ammonium salts thereof, or mixtures thereof. Preferred EDDS compounds are the free acid form and the sodium or magnesium salt thereof. Examples of such preferred sodium salts of EDDS include $Na_2$EDDS and $Na_4$EDDS. Examples of such preferred magnesium salts of EDDS include MgEDDS and $Mg_2$EDDS. The magnesium salts are the most preferred for inclusion in compositions in accordance with the invention.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated form each other by not more than two carbon atoms.

Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in amounts of from 5% to 80% by weight of the composition. Preferred levels of builder for liquid detergents are from 5% to 30%. Bleaching agents: Additional optional detergent ingredients that can be included in the detergent compositions of the present invention include bleaching agents such as perborate PB1, PB4 and percarbonate. These bleaching agent components can include one or more oxygen bleaching agents and, depending upon the bleaching agent chosen, one or more bleach activators. Present oxygen bleaching compounds will typically be present at levels of from about 1% to about 25%. In general, bleaching compounds are optional added components in non-liquid formulations, e.g. granular detergents.

The bleaching agent component for use herein can be any of the bleaching agents useful for detergent compositions including oxygen bleaches as well as others known in the art.

The bleaching agent suitable for the present invention can be an activated or non-activated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in US 4,483,781, EP 0 133 354 and US 4,412,934. Highly preferred bleaching agents also include 6-nonylamino-6-oxoperoxycaproic acid as described in US 4,634,551.

Another category of bleaching agents encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight. Such halogen bleaching agents are generally less preferred for use in enzymatic detergents.

The hydrogen peroxide releasing agents can be used in combination with bleach activators such as tetraacetylethylenediamine (TAED), nonanoyloxybenzenesulfonate (NOBS, described in US 4,412,934), 3,5-trimethyl-hexsanoloxybenzenesulfonate (ISONOBS, described in EP 120 591) or pentaacetylglucose (PAG), which are perhydrolyzed to form a peracid as the active bleaching species, leading to improved bleaching effect. In addition, very suitable are the bleach activators C8 (6-octanamido-caproyl) oxybenzene-sulfonate, C9(6-nonanamido caproyl) oxybenzenesulfonate and C10 (6-decanamido caproyl) oxybenzene-sulfonate or mixtures thereof. Also suitable activators are acylated citrate esters such as disclosed in European Patent Application No. 91870207.7.

Useful bleaching agents, including peroxyacids and bleaching systems comprising bleach activators and peroxygen bleaching compounds for use in cleaning compositions according to the invention are described in application USSN 08/136,626.

The hydrogen peroxide may also be present by adding an enzymatic system (i.e. an enzyme and a substrate therefore) which is capable of generation of hydrogen peroxide at the beginning or during the washing and/or rinsing process. Such enzymatic systems are disclosed in European Patent Application EP 0 537 381.

Bleaching agents other than oxygen bleaching agents are also known in the art and can be utilized herein. One type of non-oxygen bleaching agent of particular interest includes photoactivated bleaching agents such as the sulfonated zinc and/or aluminium phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in US 4,033,718. Typically, detergent composition will contain about 0.025% to about 1.25%, by weight, of sulfonated zinc phthalocyanine.

Bleaching agents may also comprise a manganese catalyst. The manganese catalyst may, e.g., be one of the compounds described in "Efficient manganese catalysts for low-temperature bleaching", Nature (369) 1994, pp. 637-639.

Suds suppressors: Another optional ingredient is a suds suppressor, exemplified by silicones, and silica-silicone mixtures. Silicones can generally be represented by alkylated polysiloxane materials, while silica is normally used in finely divided forms exemplified by silica aerogels and xerogels and hydrophobic silicas of various types. Theses materials can be incorporated as particulates, in which the suds suppressor is advantageously releasably incorporated in a water-soluble or water-dispersible, substantially non surface-active detergent impermeable carrier. Alternatively the suds suppressor can be dissolved or dispersed in a liquid carrier and applied by spraying onto one or more of the other components.

A preferred silicone suds controlling agent is disclosed in US 3,933,672. Other particularly useful suds suppressors are the self-emulsifying silicone suds suppressors, described in German Patent Application DTOS 2,646,126. An example of such a compound is DC-544, commercially available form Dow Corning, which is a siloxane-glycol copolymer. Especially preferred suds controlling agent are the suds suppressor system comprising a mixture of silicone

oils and 2-alkyl-alkanols. Suitable 2-alkyl-alkanols are 2-butyl-octanols, which are commercially available under the trade name Isofol 12 R.

Such suds suppressor system are described in European Patent Application EP 0 593 841 .

Especially preferred silicone suds controlling agents are described in European Patent Application No. 92201649.8. Said compositions can comprise a silicone/ silica mixture in combination with fumed nonporous silica such as Aerosil®.

The suds suppressors described above are normally employed at levels of from 0.001% to 2% by weight of the composition, preferably from 0.01 % to 1% by weight.

Other components: Other components used in detergent compositions may be employed such as soil-suspending or anti-redeposition agents, soil-releasing agents, optical brighteners, abrasives, bactericides, tarnish inhibitors, coloring agents, and/or encapsulated or non-encapsulated perfumes.

Especially suitable encapsulating materials are water soluble capsules which consist of a matrix of polysaccharide and polyhydroxy compounds such as described in GB 1,464,616.

Other suitable water soluble encapsulating materials comprise dextrins derived from ungelatinized starch acid esters of substituted dicarboxylic acids such as described in US 3,455,838. These acid-ester dextrins are, preferably, prepared from such starches as waxy maize, waxy sorghum, sago, tapioca and potato. Suitable examples of said encapsulation materials include N-Lok manufactured by National Starch. The N-Lok encapsulating material consists of a modified maize starch and glucose. The starch is modified by adding monofunctional substituted groups such as octenyl succinic acid anhydride.

Typical anti-redeposition agents used in detergents include water-soluble, generally organic colloids, including for example the water-soluble salts of polymeric carboxylic acids such as polyacrylic acid or polymaleic acid or copolymers thereof, glue, gelatine, salts of ether carboxylic acids or ether sulfonic acids of starch or cellulose or salts of sulfuric acid esters of cellulose or starch. Water-soluble polyamides containing acidic groups are also used as anti-redeposition agent. Soluble starch preparations and other starch products than those mentioned above, for example partly hydrolyzed starch, may also be used. Sodium carboxymethyl cellulose, methyl cellulose, hydroxyethyl cellulose, methyl hydroxyethyl cellulose and mixtures thereof are preferably used. These materials are normally used at levels of from 0.05% to 10% by weight, more preferably form 0.2% to 8%, most preferably from 0.5% to 6% by weight of the composition.

Preferred optical brighteners are anionic in character, examples of which are disodium 4,4'-bis-(2-diethanolamino-4-anilino-s-triazin-6-ylamino)stilbene-2:2' disulpho-nate, disodium 4, - 4'-bis-(2-morpholino-4-anilino-s-triazin-6-ylamino-stilbene-2:2' - disulphonate, disodium 4,4' - bis-(2,4-dianilino-s-triazin-6-ylamino)stilbene-2:2' - disulpho-nate, monosodium 4',4" - bis-(2,4-dianilino-s-tri-azin-6-ylamino)stilbene-2-sulphonate, disodium 4,4'-bis-(2-anilino-4-(N-methyl-N-2-hydroxyethylamino)-s-triazin-6-ylamino) stilbene-2,2' - disulphonate, disodium 4,4' -bis-(4-phenyl-2,1,3-triazol-2-yl)-stilbene-2,2' disulphonate, disodium 4,4'bis(2-anilino-4-(1-methyl-2-hydroxyethylamino)-s-triazin-6-ylami-no)stilbene-2,2'disulphonate, sodium 2(stilbyl-4"-(naphtho-1',2':4,5)-1,2,3, - triazole-2"-sulphonate and 4,4'-bis(2-sulphostyryl)biphenyl.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric poly-carboxylate salts are valuable for improving whiteness maintenance, fabric ash deposition, and cleaning performance on clay, proteinaceous and oxidizable soils in the presence of transition metal impurities.

Soil release agents useful in compositions of the present invention are conventionally copolymers or terpolymers of terephthalic acid with ethylene glycol and/or propylene glycol units in various arrangements. Examples of such polymers are disclosed in US 4,116,885 and 4,711,730 and EP 0 272 033. A particular preferred polymer in accordance with EP 0 272 033 has the formula:

$$(CH_3(PEG)_{43})_{0.75}(POH)_{0.25}[T\text{-}PO)_{2.8}(T\text{-}PEG)_{0.4}]T(POH)_{0.25}((PEG)_{43}CH_3)_{0\cdot 75}$$

where PEG is $-(OC_2H_4)0-$, PO is $(OC_3H_6O)$ and T is $(pOOC_6H_4CO)$.

Also very useful are modified polyesters as random copolymers of dimethyl terephthaiate, dimethyl sulfoisophthalate, ethylene glycol and 1,2-propanediol, the end groups consisting primarily of sulphobenzoate and secondarily of mono esters of ethylene glycol and/or 1,2-propanediol. The target is to obtain a polymer capped at both end by sulphobenzoate groups, "primarily", in the present context most of said copolymers herein will be endcapped by sulphobenzoate groups. However, some copolymers will be less than fully capped, and therefore their end groups may consist of monoester of ethylene glycol and/or 1,2-propanediol, thereof consist "secondarily" of such species.

The selected polyesters herein contain about 46% by weight of dimethyl terephthalic acid, about 16% by weight of 1,2-propanediol, about 10% by weight ethylene glycol, about 13% by weight of dimethyl sulfobenzoic acid and about 15% by weight of sulfoisophthalic acid, and have a molecular weight of about 3.000. The polyesters and their method

of preparation are described in detail in EP 311 342.

Softening agents: Fabric softening agents can also be incorporated into laundry detergent compositions in accordance with the present invention. These agents may be inorganic or organic in type. Inorganic softening agents are exemplified by the smectite clays disclosed in GB-A-1 400898 and in US 5,019,292. Organic fabric softening agents include the water insoluble tertiary amines as disclosed in GB-A1 514 276 and EP 0 011 340 and their combination with mono $C_{12}$-$C_{14}$ quaternary ammonium salts are disclosed in EP-B-0 026 528 and di-long-chain amides as disclosed in EP 0 242 919. Other useful organic ingredients of fabric softening systems include high molecular weight polyethylene oxide materials as disclosed in EP 0 299 575 and 0 313 146.

Levels of smectite clay are normally in the range from 5% to 15%, more preferably from 8% to 12% by weight, with the material being added as a dry mixed component to the remainder of the formulation. Organic fabric softening agents such as the water-insoluble tertiary amines or di-long chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

Polymeric dye-transfer inhibiting agents: The detergent compositions according to the present invention may also comprise from 0.001% to 10%, preferably from 0.01% to 2%, more preferably form 0.05% to 1% by weight of polymeric dye- transfer inhibiting agents. Said polymeric dye-transfer inhibiting agents are normally incorporated into detergent compositions in order to inhibit the transfer of dyes from colored fabrics onto fabrics washed therewith. These polymers have the ability of complexing or adsorbing the fugitive dyes washed out of dyed fabrics before the dyes have the opportunity to become attached to other articles in the wash.

Especially suitable polymeric dye-transfer inhibiting agents are polyamine N-oxide polymers, copolymers of N-vinyl-pyrrolidone and N-vinylimidazole, polyvinylpyrrolidone polymers, polyvinyloxazolidones and polyvinylimidazoles or mixtures thereof.

Addition of such polymers also enhances the performance of the enzymes according the invention.

Enzymes

A detergent composition of the invention may in an embodiment of the invention besides the endo-glucanase having anti-redeposition effect as defined above, comprise other enzyme(s) which provides cleaning performance and/or fabric care benefits.

Such enzymes include certain proteases, lipases, cutinases, cellulases, amylases, peroxidases, oxidases (e.g. laccases), and hemicellulases such as mannanase and pectate lyase.

Proteases: Any protease suitable for use in alkaline solutions can be used. Suitable proteases include those of animal, vegetable or microbial origin. Microbial origin is preferred. Chemically or genetically modified mutants are included. The protease may be a serine protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270.

Preferred commercially available protease enzymes include those sold under the trade names Everlase™, Kannase™, Alcalase™, Savinase™, Primase™, Durazym™, and Esperase™ by Novozymes A/S (Denmark), those sold under the tradename Maxatase, Maxacal, Maxapem, Properase, Purafect and Purafect OXP by Genencor International, and those sold under the tradename Opticlean and Optimase by Solvay Enzymes. Protease enzymes may be incorporated into the compositions in accordance with the invention at a level of from 0.00000 1 % to 2% of enzyme protein by weight of the composition, preferably at a level of from 0.00001% to 1% of enzyme protein by weight of the composition, more preferably at a level of from 0.0001 % to 0.5% of enzyme protein by weight of the composition, even more preferably at a level of from 0.001 % to 0.2% of enzyme protein by weight of the composition.

Lipases: Any lipase suitable for use in alkaline solutions can be used. Suitable lipases include those of bacterial or fungal origin. Chemically or genetically modified mutants are included.

Examples of useful lipases include a *Humicola lanuginosa* lipase, e.g., as described in EP 258 068 and EP 305 216, a *Rhizomucor miehei* lipase, e.g., as described in EP 238 023, a *Candida* lipase, such as a *C. antarctica* lipase, e.g., the *C. antarctica* lipase A or B described in EP 214 761, a *Pseudomonas* lipase such as a *P. alcaligenes* and *P. pseudoalcaligenes* lipase, e.g., as described in EP 218 272, a *P. cepacia* lipase, e.g., as described in EP 331 376, a *P. stutzeri* lipase, e.g., as disclosed in GB 1,372,034, a *P. fluorescens* lipase, a *Bacillus* lipase, e.g., a *B. subtilis* lipase (Dartois et al., (1993), Biochemica et Biophysica acta 1131, 253-260), a *B. stearothermophilus* lipase (JP 64/744992) and a *B. pumilus* lipase (WO 91/16422).

Furthermore, a number of cloned lipases may be useful, including the *Penicillium camembertii* lipase described

by Yamaguchi et al., (1991), Gene 103, 61-67), the *Geotricum candidum* lipase (Schimada, Y. et al., (1989), J. Biochem., 106, 383-388), and various *Rhizopus* lipases such as a *R. delemar* lipase (Hass, M.J et al., (1991), Gene 109, 117-113), a *R. niveus* lipase (Kugimiya et al., (1992), Biosci. Biotech. Biochem. 56, 716-719) and a *R. oryzae* lipase.

Other types of lipolytic enzymes such as cutinases may also be useful, e.g., a cutinase derived from *Pseudomonas mendocina* as described in WO 88/09367, or a cutinase derived from *Fusarium solani pisi* (e.g. described in WO 90/09446).

In a preferred embodiment the lipase is a variant of *Humicola lanuginosa* DSM 4109 as described in WO 00/60063. Especially preferred are the variants disclosed in the Example in WO 00/60063 with improved first wash performance., i.e., T231R+N233R; G91A+D96W+E99K+G263Q+L264A+I265T+G266D+T267A+L269N+270AGGFSWRRYRSAESVDKRATMTDAELEKKLNSYVQMDKEYVKNNQARS; R209P+T231 R+N233R; N33Q+D96S+T231 R+N233R+Q249R; E99N+N101S+T231R+ N233R+Q249R; E99N+N101S+T231R+N233R+Q249R.

Especially suitable lipases are lipases such as M1 Lipase™ and Lipomax™ (Genencor), Lipolase™ and Lipolase Ultra™, Lipex™ (Novozymes A/S), and Lipase P "Amano" (Amano Pharmaceutical Co. Ltd.). Suitable cutinases include Lumafast™ available from Genencor Inc.

The lipases are normally incorporated in the detergent composition at a level of from 0.000001 % to 2% of enzyme protein by weight of the composition, preferably at a level of from 0.00001 % to 1% of enzyme protein by weight of the composition, more preferably at a level of from 0.0001% to 0.5% of enzyme protein by weight of the composition, even more preferably at a level of from 0.001 % to 0.2% of enzyme protein by weight of the composition.

Amylases: Any amylase (alpha and/or beta) suitable for use in alkaline solutions can be used. Suitable amylases include those of bacterial or fungal origin. Chemically or genetically modified mutants are included. Amylases include, for example, alpha-amylases obtained from a special strain of *B. licheniformis,* described in more detail in GB 1,296,839. Commercially available amylases are Natalase™, Termamyl™ Ultra, Duramyl™, Termamyl™, Fungamyl™ and BAN™ (available from Novozymes A/S) and Rapidase™ and Maxamyl P™ (available from Genencor Inc.).

In a preferred embodiment the alpha-amylase is derived from *Bacillus* sp. strains NCIB 12289, NCIB 12512, NCIB 12513 and DSM 9375. Especially preferred are the alpha-amylases shown in SEQ ID NOS 1 and 2 of WO 95/26397.

In another preferred embodiment the alpha-amylase is the AA560 alpha-amylase derived from *Bacillus* sp. DSM 12649 disclosed as SEQ ID NO: 2 in WO 00/60060 . Especially preferred are variants of the AA560 alpha-amylase, including the AA560 variant disclosed in Example 7 and 8.

The amylases are normally incorporated in the detergent composition at a level of from 0.000001% to 2% of enzyme protein by weight of the composition, preferably at a level of from 0.00001% to 1% of enzyme protein by weight of the composition, more preferably at a level of from 0.0001% to 0.5% of enzyme protein by weight of the composition, even more preferably at a level of from 0.001% to 0.2% of enzyme protein by weight of the composition.

Cellulases: Any cellulase suitable for use in alkaline solutions can be used. Suitable cellulases include those of bacterial or fungal origin. Chemically or genetically modified mutants are included. Suitable cellulases are disclosed in US 4,435,307, which discloses fungal cellulases produced from *Humicola insolens.* Especially suitable cellulases are the cellulases having colour care benefits. Examples of such cellulases are cellulases described in European patent application No. 0 495 257.

In a preferred embodiment the cellulase is a *Thielavia terrestris* cellulase, preferably the cellulase disclosed in SEQ ID NO: 9 in WO 96/29397 and SEQ ID NO: 4 herein or an enzyme with at least 70% identity thereto. In a preferred embodiment cellulase is the *Thielavia terrestris* variant disclosed in Example 1 of WO 98/12307.

Commercially available cellulases include Celluzyme® produced by a strain of *Humicola insolens,* Carezyme® and Renozyme® (Novozymes A/S), and KAC-500(B)™ (Kao Corporation). Cellulases are normally incorporated in the detergent composition at a level of from 0.000001% to 2% of enzyme protein by weight of the composition, preferably at a level of from 0.00001% to 1% of enzyme protein by weight of the composition, more preferably at a level of from 0.0001% to 0.5% of enzyme protein by weight of the composition, even more preferably at a level of from 0.001 % to 0.2% of enzyme protein by weight of the composition.

Mannanases: Any mannanase suitable for use in alkaline solutions can be used. Suitable mannanases include those of bacterial or fungal origin. Chemically or genetically modified mutants are included.

In a preferred embodiment the mannanase is derived from a strain of the genus Ba*cillus*, especially *Bacillus* sp. I633 disclosed in positions 31-330 of SEQ ID NO:2 or in SEQ ID NO: 5 of WO 99/64619 or *Bacillus agaradhaerens,* for example from the type strain DSM 8721. Further, suitable mannanases are Purabrite available from Genencor Inc. and Mannaway® produced by Novozymes A/S.

Pectate lyase: Any pectate lyase suitable for use in alkaline solutions can be used. Suitable pectate lyases include those of bacterial or fungal origin. Chemically or genetically modified mutants are included.

In a preferred embodiment the pectate lyase is derived from a strain of the genus *Bacillus,* especially a strain of *Bacillus subtilis,* especially *Bacillus subtilis* DSM14218 disclosed in SEQ ID NO:2 or a variant thereof disclosed in Example 6 of WO 02/092741. Peroxidases/Oxidases: Peroxidase enzymes are used in combination with hydrogen

peroxide or a source thereof (e.g. a percarbonate, perborate or persulfate). Oxidase enzymes are used in combination with oxygen. Both types of enzymes are used for "solution bleaching", i.e. to prevent transfer of a textile dye from a dyed fabric to another fabric when said fabrics are washed together in a wash liquor, preferably together with an enhancing agent as described in e.g. WO 94/12621 and WO 95/01426. Suitable peroxidases/oxidases include those of plant, bacterial or fungal origin. Chemically or genetically modified mutants are included.

Peroxidase and/or oxidase enzymes are normally incorporated in the detergent composition at a level of from 0.000001% to 2% of enzyme protein by weight of the composition, preferably at a level of from 0.00001 % to 1 % of enzyme protein by weight of the composition, more preferably at a level of from 0.0001 % to 0.5% of enzyme protein by weight of the composition, even more preferably at a level of from 0.001 % to 0.2% of enzyme protein by weight of the composition.

Mixtures of the above mentioned enzymes are encompassed herein, in particular a mixture of two, three, four, five, six or more different enzymes, for example a protease, an amylase, a lipase and a cellulase.

The enzyme of the invention, or any other enzyme incorporated in the detergent composition, is normally incorporated in the detergent composition at a level from 0.000001 % to 2% of enzyme protein by weight of the composition, preferably at a level from 0.00001% to 1% of enzyme protein by weight of the composition, more preferably at a level from 0.0001% to 0.5% of enzyme protein by weight of the composition, even more preferably at a level from 0.001% to 0.2% of enzyme protein by weight of the composition.

Enzymes may be incorporated in the form of liquid solutions or granulates. Non-dusting granulates may be produced, e.g., as disclosed in US 4,106,991 and 4,661,452 (both to Novo Industri A/S, now Novozymes A/S) and may optionally be coated by methods known in the art. Examples of waxy coating materials are poly(ethylene oxide) products (polyethyleneglycol, PEG) with mean molecular weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591.

Detergent composition examples: The detergent composition according to the invention can be in liquid, paste, gels, bars, tablet or granular forms.

Granular compositions according to the present invention can also be in "compact form", i.e. they may have a relatively higher density than conventional granular detergents, i.e. form 550 to 950 g/l; in such case, the granular detergent compositions according to the present invention will contain a lower amount of "Inorganic filler salt", compared to conventional granular detergents; typical filler salts are alkaline earth metal salts of sulphates and chlorides, typically sodium sulphate; "Compact" detergent typically comprise not more than 10% filler salt. The liquid compositions according to the present invention can also be in "concentrated form", in such case, the liquid detergent compositions according to the present invention will contain a lower amount of water, compared to conventional liquid detergents. Typically, the water content of the concentrated liquid detergent is less than 30%, more preferably less than 20%, most preferably less than 10% by weight of the detergent compositions.

The compositions of the invention may for example, be formulated as hand and machine laundry detergent compositions including laundry additive compositions and compositions suitable for use in the pretreatment of stained fabrics, rinse added fabric softener compositions, and compositions for use in general household hard surface cleaning operations and machine or hand dishwashing operations.

The following examples are meant to exemplify compositions for the present invention, but are not necessarily meant to limit or otherwise define the scope of the invention.

In the detergent compositions, the abbreviated component identifications have the following meanings:

| | |
|---|---|
| LAS: | Sodium linear $C_{12}$ alkyl benzene sulphonate |
| TAS: | Sodium tallow alkyl sulphate |
| XYAS: | Sodium $C_{1X}$ - $C_{1Y}$ alkyl sulfate |
| SS: | Secondary soap surfactant of formula 2-butyl octanoic acid |
| 25EY: | A $C_{12}$ - $C_{15}$ predominantly linear primary alcohol condensed with an average of Y moles of ethylene oxide |
| 45EY: | A $C_{14}$ - $C_{15}$ predominantly linear primary alcohol condensed with an average of Y moles of ethylene oxide |
| XYEZS: | $C_{1X}$ - $C_{1Y}$ sodium alkyl sulfate condensed with an average of Z moles of ethylene oxide |

| | |
|---|---|
| | per mole |
| Nonionic: | $C_{13}$ - $C_{15}$ mixed ethoxylated/propoxylated fatty alcohol with an average degree of ethoxylation of 3.8 and an average degree of propoxylation of 4.5 sold under the tradename Plurafax LF404 by BASF Gmbh |
| CFAA: | $C_{12}$ - $C_{14}$ alkyl N-methyl glucamide |
| TFAA: | $C_{16}$ - $C_{18}$ alkyl N-methyl glucamide |
| Silicate: | Amorphous Sodium Silicate ($SiO_2$:$Na_2O$ ratio = 2.0) |
| NaSKS-6: | Crystalline layered silicate of formula $\delta$-$Na_2Si_2O_5$ |
| Carbonate: | Anhydrous sodium carbonate |
| Phosphate: | Sodium tripolyphosphate |
| MA/AA: | Copolymer of 1:4 maleic/acrylic acid, average molecular weight about 80,000 |
| Polyacrylate: | Polyacrylate homopolymer with an average molecular weight of 8,000 sold under the tradename Sokalan PA30 by BASF Gmbh |
| Zeolite A: | Hydrated Sodium Aluminosilicate of formula $Na_{12}(AlO_2SiO_2)_{12}.27H_2O$ having a primary particle size in the range from 1 to 10 micrometers |
| Citrate: | Tri-sodium citrate dihydrate |
| Citric: | Citric Acid |
| Perborate: | Anhydrous sodium perborate monohydrate bleach, empirical formula $NaBO_2.H_2O_2$ |
| PB4: | Anhydrous sodium perborate tetrahydrate |
| Percarbonate: | Anhydrous sodium percarbonate bleach of empirical formula $2Na_2CO_3.3H_2O_2$ |
| TAED: | Tetraacetyl ethylene diamine |
| CMC: | Sodium carboxymethyl cellulose |
| DETPMP: | Diethylene triamine penta (methylene phosphonic acid), marketed by Monsanto under the Tradename Dequest 2060 |
| PVP: | Polyvinylpyrrolidone polymer |
| EDDS: | Ethylenediamine-N, N'-disuccinic acid, [S,S] isomer in the form of the sodium salt |
| Suds Suppressor: | 25% paraffin wax Mpt 50°C, 17% hydrophobic silica, 58% paraffin oil |
| Granular Suds suppressor: | 12% Silicone/silica, 18% stearyl alcohol, 70% starch in granular form |
| Sulphate: | Anhydrous sodium sulphate |
| HMWPEO: | High molecular weight polyethylene oxide |
| TAE 25: | Tallow alcohol ethoxylate (25) |

Detergent Example I

A granular fabric cleaning composition in accordance with the invention may be prepared as follows:

| | |
|---|---|
| Sodium linear $C_{12}$ alkyl benzene sulfonate | 6.5 |
| Sodium sulfate | 15.0 |
| Zeolite A | 26.0 |
| Sodium nitrilotriacetate | 5.0 |
| Enzymes (incl. endoglucanase) | 0.1 |
| PVP | 0.5 |
| TAED | 3.0 |
| Boric acid | 4.0 |
| Perborate | 18.0 |
| Phenol sulphonate | 0.1 |
| Minors | Up to 100 |

Detergent Example II

A compact granular fabric cleaning composition (density 800 g/l) in accord with the invention may be prepared as follows:

| | |
|---|---|
| 45AS | 8.0 |
| 25E3S | 2.0 |
| 25E5 | 3.0 |
| 25E3 | 3.0 |
| TFAA | 2.5 |
| Zeolite A | 17.0 |
| NaSKS-6 | 12.0 |
| Citric acid | 3.0 |
| Carbonate | 7.0 |
| MA/AA | 5.0 |
| CMC | 0.4 |
| Enzyme (incl. endo-glucanase) | 0.1 |
| TAED | 6.0 |
| Percarbonate | 22.0 |
| EDDS | 0.3 |
| Granular suds suppressor | 3.5 |
| water/minors | Up to 100% |

Detergent Example III

Granular fabric cleaning compositions in accordance with the invention which are especially useful in the laundering of coloured fabrics were prepared as follows:

| | | |
|---|---|---|
| LAS | 10.7 | - |
| TAS | 2.4 | - |
| TFAA | - | 4.0 |
| 45AS | 3.1 | 10.0 |
| 45E7 | 4.0 | - |
| 25E3S | - | 3.0 |
| 68E11 | 1.8 | - |
| 25E5 | - | 8.0 |

(continued)

| | | |
|---|---|---|
| Citrate | 15.0 | 7.0 |
| Carbonate | - | 10 |
| Citric acid | 2.5 | 3.0 |
| Zeolite A | 32.1 | 25.0 |
| Na-SKS-6 | - | 9.0 |
| MA/AA | 5.0 | 5.0 |
| DETPMP | 0.2 | 0.8 |
| Enzyme(incl. endo-glucanase) | 0.10 | 0.05 |
| Silicate | 2.5 | - |
| Sulphate | 5.2 | 3.0 |
| PVP | 0.5 | - |
| Poly (4-vinylpyridine)-N-Oxide/copolymer of vinylimidazole and vinylpyrrolidone | - | 0.2 |
| Perborate | 1.0 | - |
| Phenol sulfonate | 0.2 | - |
| Water/Minors | Up to 100% | |

Detergent Example IV

Granular fabric cleaning compositions in accordance with the invention which provide "Softening through the wash" capability may be prepared as follows:

| | | |
|---|---|---|
| 45AS | - | 10.0 |
| LAS | 7.6 | - |
| 68AS | 1.3 | - |
| 45E7 | 4.0 | - |
| 25E3 | - | 5.0 |
| Coco-alkyl-dimethyl hydroxy-ethyl ammonium chloride | 1.4 | 1.0 |
| Citrate | 5.0 | 3.0 |
| Na-SKS-6 | - | 11.0 |
| Zeolite A | 15.0 | 15.0 |
| MA/AA | 4.0 | 4.0 |
| DETPMP | 0.4 | 0.4 |
| Perborate | 15.0 | - |
| Percarbonate | - | 15.0 |
| TAED | 5.0 | 5.0 |
| Smectite clay | 10.0 | 10.0 |
| HMWPEO | - | 0.1 |
| Enzyme (incl. endo-glucanase) | 0.10 | 0.05 |
| Silicate | 3.0 | 5.0 |
| Carbonate | 10.0 | 10.0 |
| Granular suds suppressor | 1.0 | 4.0 |
| CMC | 0.2 | 0.1 |
| Water/Minors | Up to 100% | |

Detergent Example V

Heavy duty liquid fabric cleaning compositions in accordance with the invention may be prepared as follows:

| | I | II |
|---|---|---|
| LAS acid form | - | 25.0 |
| Citric acid | 5.0 | 2.0 |
| 25AS acid form | 8.0 | - |
| 25AE2S acid form | 3.0 | - |
| 25AE7 | 8.0 | - |
| CFAA | 5 | - |
| DETPMP | 1.0 | 1.0 |
| Fatty acid | 8 | - |
| Oleic acid | - | 1.0 |
| Ethanol | 4.0 | 6.0 |
| Propanediol | 2.0 | 6.0 |
| Enzyme (incl.endo-glucanase) | 0.10 | 0.05 |
| Coco-alkyl dimethyl hydroxy ethyl ammonium chloride | - | 3.0 |
| Smectite clay | - | 5.0 |
| PVP | 2.0 | - |
| Water / Minors | Up to 100% | |

Use of detergents: The enzyme composition of the invention may be useful in a detergent composition for household or industrial laundering of textiles and garments, and in a process for machine wash treatment of fabrics comprising treating the fabrics during one or more washing cycle of a machine washing process with a washing solution containing the enzyme or enzyme preparation of the invention.

The enzyme composition of the invention may also be useful in a detergent composition for household or industrial dish or cutlery or other hard surface washing, and in a process for treatment of dishes, cutlery etc. comprising a treatment with a washing solution containing the enzyme or enzyme preparation of the invention.

Typically, the detergent composition used in the washing process comprises conventional ingredients such as surfactants (anionic, nonionic, zwitterionic, amphoteric), builders, bleaches (perborates, percarbonates or hydrogen peroxide) and other ingredients, e.g. as described in WO 97/01629.

The endo-glucanase of the invention provides advantages such as improved stain removal and decreased soil redeposition. Certain stains, for example certain food stains, contain beta-glucans which make complete removal of the stain difficult to achieve. Also, the cellulosic fibres of the fabrics may possess, particularly in the "non-crystalline" and surface regions, glucan polymers that are degraded by this enzyme. Hydrolysis of such glucans, either in the stain or on the fabric, during the washing process decreases the binding of soils onto the fabrics.

Household laundry processes are carried out under a range of conditions. Commonly, the washing time is from 5 to 60 minutes and the washing temperature is in the range 10 - 60°C, most commonly from 20 - 40°C. Prolonged soaking is commonly used. The washing solution is normally neutral or alkaline, most commonly with pH 5 - 11.5. Bleaches are commonly used, particularly for laundry of white fabrics and in the washing of hard surfaces. These bleaches are commonly the peroxide bleaches, such as sodium perborate, sodium percarbonate or hydrogen peroxide.

## MATERIALS & METHODS

### Strains and donor organism

The *Bacillus sp.* DSM 12648 mentioned above comprises the endo-glucanase encoding DNA sequence shown in SEQ ID NO:1.

*B.subtilis* PL2306: This strain is the *B.subtilis* DN1885 with disrupted apr and npr genes (Diderichsen, B., Wedsted, U., Hedegaard, L., Jensen, B. R., Sjøholm, C. (1990) Cloning of aldB, which encodes alpha-acetolactate decarboxylase, an exoenzyme from Bacillus brevis. J. Bacteriol., 172, 4315-4321) disrupted in the transcriptional unit of the known *Bacillus subtilis* cellulase gene, resulting in cellulase negative cells. The disruption was performed essentially as described in Eds. A.L. Sonenshein, J.A. Hoch and Richard Losick (1993) Bacillus subtilis and other Gram-Positive Bacteria, American Society for microbiology, p.618.

Competent cells were prepared and transformed as described by Yasbin, R.E., Wilson, G.A. and Young, F.E. (1975) Transformation and transfection in lysogenic strains of Bacillus subtilis: evidence for selective induction of

prophage in competent cells. J. Bacteriol, 121:296-304.

## General molecular biology methods

Unless otherwise stated all the DNA manipulations and transformations were performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).

Enzymes for DNA manipulations were used according to the manufacturer's instructions (e.g. restriction endonucleases, ligases etc. are obtainable from New England Biolabs, Inc.).

## Plasmids

**pMOL944.** This plasmid is a pUB110 derivative essentially containing elements making the plasmid propagate in *Bacillus subtilis,* kanamycin resistance gene and having a strong promoter and signal peptide cloned from the amyL gene of *B.licheniformis* ATCC14580. The signal peptide contains a SacII site making it convenient to clone the DNA encoding the mature part of a protein in-fusion with the signal peptide. This results in the expression of a Pre-protein which is directed towards the exterior of the cell.

The plasmid was constructed by means of ordinary genetic engineering and is briefly described in the following.

Construction of pMOL944:

The pUB110 plasmid (McKenzie, T. et al., 1986, Plasmid 15:93-103) was digested with the unique restriction enzyme NciI. A PCR fragment amplified from the amyL promoter encoded on the plasmid pDN1981 (P.L. Jørgensen et al., 1990, Gene, 96, p37-41.) was digested with NciI and inserted in the NciI digested pUB110 to give the plasmid pSJ2624.

The two PCR primers used have the following sequences:

# LWN5494 (SEQ ID NO:5)
5'-GTCGCCGGGGCGGCCGCTATCAATTGGTAACTGTATCTCAGC -3'
# LWN5495 (SEQ ID NO:6)

5´-GTCGCCCGGGAGCTCTGATCAGGTACCAAGCTTGTCGACCTGCAGAA
TGAGGCAGCAAGAAGAT -3´

The primer #LWN5494 inserts a NotI site in the plasmid.

The plasmid pSJ2624 was then digested with SacI and NotI and a new PCR fragment amplified on amyL promoter encoded on the pDN1981 was digested with SacI and NotI and this DNA fragment was inserted in the SacI-NotI digested pSJ2624 to give the plasmid pSJ2670.

This cloning replaces the first amyL promoter cloning with the same promoter but in the opposite direction. The two primers used for PCR amplification have the following sequences:

#LWN5938 (SEQ ID NO:7)

5`-GTCGGCGGCCGCTGATCACGTACCAAGCTTGTCGACCTGCAGAATG
AGGCAGCAAGAAGAT -3´

#LWN5939 (SEQ ID NO:8)
5'-GTCGGAGCTCTATCAATTGGTAACTGTATCTCAGC -3'

The plasmid pSJ2670 was digested with the restriction enzymes PstI and BclI and a PCR fragment amplified from a cloned DNA sequence encoding the alkaline amylase SP722 (Patent # WO9526397-A1) was digested with PstI and BclI and inserted to give the plasmid pMOL944. The two primers used for PCR amplification have the following sequence:

#LWN7864 (SEQ ID NO:9)
5'-AACAGCTGATCACGACTGATCTTTTAGCTTGGCAC-3'
#LWN7901 (SEQ ID NO:10)
5' -AACTGCAGCCGCGGCACATCATAATGGGACAAATGGG -3'

The primer #LWN7901 inserts a SacII site in the plasmid.

**Genomic DNA Preparation**

The strain DSM 12648 was propagated in liquid medium 2xTY containing 1% carboxymethyl-cellulose + (0.1 M Na2CO3 + 0.1 M NaHCO3 separately autoclaved and added aseptically after cooling to room temperature). After 16 hours of incubation at 30°C and 300 rpm, the cells were harvested, and genomic DNA was isolated by the method described by *Pitcher et al.* [Pitcher, D. G., Saunders, N. A., Owen, R. J; Rapid extraction of bacterial genomic DNA with guanidium thiocyanate; Lett Appl Microbiol 1989, 8:151-156].

**Media**

**TY** (as described in Ausubel, F. M. et al. (eds.): "Current protocols in Molecular Biology", John Wiley and Sons, 1995).
**2xTY** (as described in Ausubel, F. M. et al. (eds.): "Current protocols in Molecular Biology", John Wiley and Sons, 1995).
**LB agar** (as described in Ausubel, F. M. et al. (eds.): "Current protocols in Molecular Biology", John Wiley and Sons, 1995).
**LBPG** is LB agar supplemented with 0.5% Glucose and 0.05 M potassium phosphate, pH 7.0
**AZCL-HE-cellulose** is added to LBPG-agar to 0.5 % AZCL- HE-cellulose is from Megazyme, Australia.
**BPX media** is described in EP 0 506 780 (WO 91/09129).
**Cal 18-2 media** is described in patent application WO 00/75344 A1).

**<u>Test for endo-glucanase activity</u>**

The purpose of this test, used in combination with the "test for anti-redeposition effect" described below, is to determine if an enzyme is an endo-glucanase with anti-redeposition effect, according to this invention.
The test is made by determining the color released from an insoluble, colored, glucan substrate over a reaction time of 30 minutes at a temperature of 40°C.
Enzyme sample: The enzyme sample for testing is a solution of the enzyme protein with a concentration of 0.1 mg /ml. Standard biochemical techniques can be used to verify the purity and determine the protein concentration of the sample.
Buffer: Prepare a 0.05M phosphate buffer solution with pH 7.0 from $NaH_2PO_4.2H_2O$ and NaOH and add 1g/l nonionic surfactant (e.g. Berol 537, from Akzo Nobel).
Test method: Transfer 6 ml buffer into two test tubes. To one tube add 50µl of the enzyme sample. No enzyme is added to the second tube. Add one beta-glucazyme tablet (supplied by Megazyme, Ireland, catalogue number T-BGZ) to each tube. Mix the contents of the tubes for 10 seconds with a vortex mixer, then place the tubes in a 40°C water bath. After 10 minutes and after 20 minutes, mix the contents of the tubes by inverting the tubes. After 30 minutes, mix the contents of the tubes by inverting the tubes and then filter the contents of the tubes through a Whatman GF/C 9cm filter, collecting the filtrates in clean tubes. Measure the color released as OD at 590 nm using a spectrophotometer. Calculate deltaOD by subtracting the result with no enzyme from the result with enzyme.
If deltaOD > 0.2 then the enzyme is an endo-glucanase.

**<u>Test for anti-redeposition effect</u>**

The purpose of this test, used in combination with the "test for endo-glucanase activity" described above, is to determine if an enzyme is an endo-glucanase with anti-redeposition effect, according to this invention.
The anti-redeposition effect is measured by a wash test. The anti-redeposition wash test is made by washing samples of soiled cotton fabric and samples of clean cotton fabric, both together, in a small-scale wash test apparatus. After the washing the soil on the originally clean cotton fabric is evaluated by light reflectance.
Enzyme sample: The enzyme sample for testing is a solution of the enzyme protein with a concentration of 0.1 mg /ml. Standard biochemical techniques can be used to verify the purity and determine the protein concentration of the sample.

Cotton fabric: #2003 white woven 100% cotton fabric, supplied by Tanigashira, 4-11-15 Komatsu Yodogawa-ku, Osaka, 533-0004, Japan. The new cotton fabric is pre-washed three times before use in the wash test. The pre-washing is done using a European household front-loader washing machine, and using a standard 40°C wash process. LAS (Surfac® SDBS80 sodium alkylbenzene sulfonate, 80%) is added to the wash water at concentration 0.5 g per liter and the wash solution pH is adjusted to 10 by addition of sodium carbonate. After the pre-washing the fabric is dried in a tumbler drier. Swatches of the pre-washed cotton fabric, size 5x5cm, weight approximately 0.3g each, are cut out and these swatches are used for the wash tests.

Soiled cotton swatches: Swatches of the pre-washed #2003 fabric, prepared as above, are soiled as follows. A suspension of 210 mg carbon black ("carbon for detergency tests", supplied by Sentakukagaku-kyokai, 2-11-1 Shimomaruko Ohta-ku, Tokyo 146-8620, Japan) is prepared in 75 ml tetrachloroethylene (Fluka, cat. nr. 86972) by strong stirring. The cotton swatches are placed flat on a horizontal metal surface. 300μl of the carbon suspension is pipetted onto the centre of each cotton swatch. The soiled cotton swatches are allowed to dry at room temperature overnight.

Detergent solutions: Detergent solutions are prepared as follows. To prepare 1 liter of solution, dissolve in deionised water 0.5g sodium carbonate and 1.0g sodium hydrogen carbonate and add 2 ml of a solution containing 117.8 g/l $CaCl_2.2H_2O$ and 54.3 g/l $MgCl_2.6H_20$. This calcium/magnesium addition provides a water hardness of 12 °dH. Add 0.5g LAS (Surfac SDBS80 sodium alkylbenzene sulfonate, 80%, supplied by Surfachem, UK or an equivalent material) and adjust the final volume to 1 liter. Adjust the pH to 9.5 (e.g. by addition of sodium carbonate)

Wash tests: Three soiled swatches (prepared as described) and three clean swatches (of the same pre-washed #2003 cotton) are washed in a Mini-Terg-O-Tometer machine. The Mini-Terg-O-Tometer is a small-scale version of the Terg-O-Tometer test washing machine described in Jay C. Harris, "Detergency Evaluation and Testing", Interscience Publishers Ltd. (1954) pp. 60-61. The following conditions are used:

| | |
|---|---|
| Beaker size | 250ml |
| Wash solution volume | 100ml |
| Wash temperature | 40°C |
| Wash time | 30 minutes |
| Agitation | 150 rpm |

The detergent solutions are pre-warmed to 40°C before starting the test. The cotton swatches and 100μl of the enzyme sample are added at the start of the 30 minute wash period. The reference test, with no added enzyme, is started at the same time.

After the wash, the fabric swatches are rinsed for 5 minutes under running tap water, then spread out flat and allowed to air dry at room temperature overnight.

Instrumental evaluations: Light reflectance evaluation of the originally clean fabric swatches is done using a Macbeth Color Eye 7000 reflectance spectrophotometer. The measurements are made at 500nm. The UV filter is not included. Measurements are made on the front and back of each swatch. An average result for reflectance (R, 500nm) for the three originally clean swatches is then calculated from the six measurements.

The anti-redeposition effect of a given enzyme sample is calculated as:

$$(\text{R, 500nm with the enzyme}) - (\text{R, 500nm no enzyme added})$$

The enzyme is an endo-glucanase with anti-redeposition effect according to the invention if the anti-redeposition effect obtained is >7.5, <u>and</u> the result in the test for endo-glucanase activity, above, is >0.2.

**<u>LAS stability test</u>**

The test is made by comparing the color released from an insoluble colored cellulase substrate over a reaction time of 60 minutes at a temperature of 40°C when the solution contains a buffer but no surfactant and when the solution contains a buffer and LAS. If the enzyme is stable during the 60 minute reaction time then the color released is greater than if the enzyme is not stable, because the stable enzyme will continue to degrade the substrate whereas the non-stable enzyme will not continue to degrade the substrate. In the context of the present invention, if the result with LAS is > 50% of the result without LAS then the enzyme is LAS stable.

The details of the test method are as follows.
Buffer (no LAS):

Prepare a 0.05M phosphate buffer solution with pH 7.0 from $NaH_2PO_4.2H_2O$ and NaOH.

Buffer (with LAS):

Dissolve 1.0g LAS in 1 liter of the above buffer. The LAS is Surfac® SDBS80 sodium alkylbenzene sulfonate, 80% (supplied by Surfachem, UK) or an equivalent material.

Test method:

Prepare a dilution of the enzyme to be tested in deionised water. The concentration of the enzyme is such that the result for deltaOD (no LAS), calculated as described below, is in the range 0.2 to 0.5.

Transfer 6ml of the buffer (no LAS) into two test tubes. Add 150μl of the enzyme dilution into one tube. No enzyme is added to the other tube. Add one Cellazyme® C tablet (supplied by Megazyme, Ireland, catalogue number T-CCZ) into both tubes. Mix strongly for about 10 seconds using a vortex mixer. Place both tubes in a 40°C water bath. After 15, 30 and 45 minutes, mix the contents of the tubes by inverting the tubes. After 60 minutes, mix the contents of the tubes by inverting the tubes and then filter the contents through a 9cm diameter Whatman® GF/C filter, collecting the filtrate in a clean tube. Measure the OD of the filtrate at 590 nm using a spectrophotometer. Calculate deltaOD (no LAS) by subtracting the result with no enzyme from the result with enzyme.

Then repeat the test using the same concentration of the enzyme and with 6 ml buffer (with LAS) instead of 6 ml buffer (no LAS). Calculate deltaOD (with LAS) by subtracting the result with no enzyme from the result with enzyme.

Calculate the LAS stability %:

$$(\text{deltaOD (with LAS)} / \text{deltaOD (no LAS)}) * 100$$

**Wash tests with the endo-glucanase in combination with other enzymes**

This procedure is used to determine the enzyme detergency benefit values, see Examples 6 - 11 below.

The wash tests are made by washing samples of soiled cotton fabric and samples of clean cotton fabric, both together, in a small-scale wash test apparatus. After the washing the soil on the cotton fabric is evaluated by light reflectance. Both the originally soiled cotton fabric and the originally clean cotton fabric samples are evaluated.

Cotton fabric: #2003 white woven 100% cotton fabric, supplied by Tanigashira, 4-11-15 Komatsu Yodogawa-ku, Osaka, 533-0004, Japan. The new cotton fabric is pre-washed three times before use in the wash test. The pre-washing is done using a European household front-loader washing machine, and using a standard 40°C wash process. LAS (Surfac® SDBS80 sodium alkylbenzene sulfonate, 80%) is added to the wash water at concentration 0.5 g per liter and the wash solution pH is adjusted to 10 by addition of sodium carbonate. After the pre-washing the fabric is dried in a tumbler drier. Swatches of the pre-washed cotton fabric, size 5x5cm, weight approximately 0.3g each, are cut out and these swatches are used for the wash tests.

Soiled cotton swatches: These are prepared from the 5x5cm swatches described above.

Wash tests: Three soiled swatches and three clean swatches are washed in a Mini-Terg-O-Tometer machine. The Mini-Terg-O-Tometer is a small-scale version of the Terg-O-Tometer test washing machine described in Jay C. Harris, "Detergency Evaluation and Testing", Interscience Publishers Ltd. (1954) pp. 60-61. The following conditions are used:

| | |
|---|---|
| Beaker size | 250ml |
| Wash solution volume | 100ml |
| Wash temperature | 40°C |
| Wash time | 30 minutes |
| Agitation | 150 rpm |

The detergent solutions are pre-warmed to 40°C before starting the test. The fabric and the enzymes are added at the start of the 30 minute wash period. After the wash, the fabric swatches are rinsed for 5 minutes under running tap water, then spread out flat and allowed to air dry at room temperature overnight.

Instrumental evaluations: Light reflectance evaluation of the fabric swatches is done using a Macbeth Color Eye 7000 reflectance spectrophotometer. The measurements are made at 500nm. The UV filter is not included. Measurements are made on the front and back of each swatch. The soiled swatches are measured in the centre of the soiled area. Average results for reflectance (R, 500nm) for the soiled swatches and for the clean swatches are then calculated from the six measurements on each type.

Detergent solutions: Detergent solutions are prepared as follows: To prepare 1 liter of solution, dissolve in

deionised water 0.5g sodium carbonate and 1.0g sodium hydrogen carbonate and add 2 ml of a solution containing 117.8 g/l $CaCl_2.2H_2O$ and 54.3 g/l $MgCl_2.6H_2O$. This calcium/magnesium addition provides a water hardness of 12 °dH. Add nonionic surfactant (Berol® 537, Akzo Nobel) and/or LAS (Surfac® SDBS80 sodium alkylbenzene sulfonate, 80%) and adjust the final volume to 1 liter. Adjust the pH to either pH 9.5 (by addition of sodium carbonate) or to pH 7.5 (by addition of 10% citric acid solution).

The concentration of LAS and of nonionic surfactant and the detergent solution pH are specified below for each wash test.

Enzyme addition: The enzymes to be tested are pre-dissolved at known concentrations in water, and the required amount of enzyme is added to the detergent solution at the start of the wash process.

Calculation of enzyme detergency benefit: The enzyme detergency benefit is a measure of how much more clean the swatches, both the originally soiled and the originally clean, become as a result of including enzymes in the wash test. The enzyme detergency benefit is calculated as follows:

After the wash test the average R, 500nm value for the soiled swatches is R, soiled.
After the wash test the average R, 500nm value for the clean swatches is R, clean.

The enzyme detergency benefit from a wash test with enzymes is the sum of R, soiled + R, clean with enzymes minus the sum of R, soiled + R, clean with no added enzyme.

The enzyme detergency benefit value determined in this way is a combined measure both of the removal of soil from the fabric and of the redeposition of soil onto the fabric. Thus the enzyme detergency benefit value can have values that are negative or positive. The enzyme detergency benefit value can be used to compare the performance of different enzymes. The highest positive detergency benefit value is the preferred result.

## EXAMPLE 1

### Cloning and expression of endo-glucanase gene from *Bacillus sp.*

Sub-cloning and expression of mature endo-glucanase in *B.subtilis.*

The endo-glucanase encoding DNA sequence of the invention was PCR amplified using the PCR primer set consisting of these two oligo-nucleotides:

# 168684 (SEQ ID NO:11)
5'-CAT TCT GCA GCC GCG GCA GCA GAA GGA AAC ACT CGT GAA GAC-3'
# 168685 (SEQ ID NO:12)
5'-GCG TTG AGA CGC GCG GCC GCT TAC TCT TCT TTC TCT TCT TTC TC-3'

Restriction sites SacII and NotI are underlined.

The oligonucleotides were used in a PCR reaction in HiFidelity™ PCR buffer (Boehringer Mannheim, Germany) supplemented with 200 micro M of each dNTP, 2.6 units of HiFidelity™ Expand enzyme mix and 200 pmol of each primer. Chromosomal DNA isolated from *Bacillus sp.* DSM12648 as described above was used as template.

The PCR reaction was performed using a DNA thermal cycler (Landgraf®, Germany). One incubation at 94°C for 1 min followed by ten cycles of PCR performed using a cycle profile of denaturation at 94°C for 15 sec, annealing at 60°C for 60 sec, and extension at 72°C for 120sec, followed by twenty cycles of denaturation at 94°C for 15 sec, 60°C for 60 sec and 72°C for 120 sec (at this elongation step 20 sec are added every cycle). 5 μl aliquots of the amplification product was analysed by electrophoresis in 0.7 % agarose gels (NuSieve®, FMC). The appearance of a DNA fragment size 2.4 kb indicated proper amplification of the gene segment.

Subcloning of PCR fragment:

45 microL aliquots of the PCR products generated as described above were purified using QIAquick® PCR purification kit (Qiagen®, USA) according to the manufacturer's instructions. The purified DNA was eluted in 50 microL of 10mM Tris-HCl, pH 8.5.

5 μg of pMOL944 and 25 microL of the purified PCR fragment was digested with SacII and NotI, electrophoresed in 0.7 % agarose gels (NuSieve®, FMC), the relevant fragments were excised from the gels, and purified using QIAquick® Gel extraction Kit (Qiagen®, USA) according to the manufacturer's instructions. The isolated PCR DNA fragment was then ligated to the SacII-NotI digested and purified pMOL944. The ligation was performed overnight at 16°C using 0.5 micro g of each DNA fragment, 1 U of T4 DNA ligase and T4 ligase buffer (Boehringer Mannheim, Germany).

The ligation mixture was used to transform competent *B. subtilis* PL2306. The transformed cells were plated onto LBPG-10 micro g/ml of kanamycin-agar plates. After 18 hours incubation at 37°C colonies were seen on the plates. Several clones were analyzed by isolating plasmid DNA from overnight culture broths.

One such positive clone was re-streaked several times on agar plates as used above; this clone was called MB1181-7. The clone MB1181-7 was grown overnight in TY-10micro g/mL kanamycin at 37°C, and next day 1 ml of cells were used to isolate a plasmid from the cells using the Qiaprep® Spin Plasmid Miniprep Kit #27106 according to the manufacturers recommendations for *B. subtilis* plasmid preparations. This DNA was sequenced and revealed a DNA sequence identical to the endo-glucanase gene in SEQ ID NO:1 bp 1-2322 encoding the mature endo-glucanase. The derived protein sequence is represented in SEQ ID NO: 2.

## EXAMPLE 2

### Expression and recovery of the endo-glucanase from *Bacillus sp.* DSM 12648

MB1181-7 obtained as described in Example 1 was grown in 15 x 200 ml Cal-18-2 media with 10 microg/mL of kanamycin, in 500 mL two-baffled shake flasks, for 4 days at 37°C at 300 rpm, whereby about 2500 ml of culture broth was obtained. The culture fluid was flocculated by adding 50% $CaCl_2$ (10 ml per liter of culture broth) together with 11% sodium aluminate (10 mL per liter of culture broth), maintaining the pH between 7.0 and 7.5 by adding 20% formic acid. Cationic agent Superfloc® C521 (25 mL of a 10% v/v dilution per liter of culture broth) and anionic agent Superfloc® A130 (75 ml of a 0.1% w/v dilution in water per liter of culture broth) was added during agitation to complete the flocculation. The flocculated material was separated by centrifugation using a Sorval® RC 3B centrifuge at 10000 rpm for 30 min at 6°C. The resulting supernatant contained the endo-glucanase activity.

The supernatant was clarified using Whatman glass filters GF/D and C. Then ultra-filtration was used to concentrate and reduce the ionic strength of the solution. The ultra-filtration membrane was Filtron® UF with a cut-off of 10 kDa. After ultra-filtration the solution had conductivity < 3mS/cm. The pH was adjusted to pH 8.0.

Anion-exchange chromatography on Q-Sepharose® was then used for additional purification. The solution from ultra-filtration was applied to a 300 mL column containing Q-Sepharose® (Pharmacia) equilibrated with a buffer of 25 mmol Tris pH 8.0. The endo-glucanase bound to the Q-Sepharose®, and was then eluted using a 0.5 M NaCl gradient. The fractions with high endo-glucanase activity were pooled. The endo-glucanase activity of the final pooled endo-glucanase solution was approximately 1000 ECU per mL.

The activity units, ECU, are determined by method SM-0302, which is available from Novozymes A/S on request. In the ECU method the ability of the enzyme sample to reduce the viscosity of a solution of carboxymethyl-cellulose is determined, and the result is given in ECU. Conditions: CMC type 7LFD from Hercules, pH7.5 in 0.1M phosphate buffer, CMC concentration 31.1 g per liter, reaction at 40°C for 30 minutes. A vibration viscosimeter such as MIVI 3000, Sofraser®, France is used to measure the viscosity.

## EXAMPLE 3: Stain removal and anti-redeposition effect

This test demonstrates the stain removal and anti-redeposition effects of the endo-glucanase obtained in Example 2. Additionally this test demonstrates that the enzyme performance is essentially unchanged when sodium perborate bleach is included.

Cotton swatches are stained with beta-glucan (from barley) plus carbon black. Soiled swatches are washed together with clean swatches. After washing the swatches are rinsed and dried. The soil removal from the soiled switches and the soil redeposition onto the clean swatches is determined by reflectance measurements. The soil removal and soil redeposition after washing without or with addition of the endo-glucanase are compared.

Swatches: Cut from 100% cotton fabric, type #2003 (Tanigashira, Osaka, Japan), pre-washed at 40°C as a precaution to remove any water soluble contaminations, size 5x5cm, weight approximately 0.3g.

Washing equipment: Stirred beakers, beaker volume 250 ml, with temperature control by water bath heating. The equipment is the Mini-Terg-O-Tometer, a multi-beaker miniature agitator washer, as described in the Materials and Methods section.

Detergent solution: Prepared by adding the following into deionised water.

Sodium carbonate, 0.5 g per liter

Sodium bicarbonate, 0.7 g per liter

$Ca^{2+}/Mg^{2+}$, to give water hardness 12°dH

Anionic surfactant, Surfac® SDBS80 (sodium alkylbenzene sulphonate), 0.5 g per liter

Nonionic surfactant, Berol® 537 (Akzo Nobel), 1.0 g per liter

Sodium perborate, type SPB from wfk Testgewebe, either 0 or 1.0 g per liter

Solution pH is approximately 9.5.

Washing procedure: 100mL detergent solution is added to each beaker. The water bath temperature is 40°C. The mechanical agitators are operated at approximately 125 rpm. The detergent solutions are pre-warmed for 10 minutes and then the endo-glucanase and the swatches are added. In each case three soiled swatches (prepared as described below) and three clean swatches are added to each beaker. After washing for 30 minutes, the swatches are removed from the detergent solution, rinsed under running tap water for 5 minutes, spread flat on absorbent paper and allowed to dry.

Reflectance measurements: Made using a Macbeth® 7000 Color Eye reflectance spectrophotometer. In the case of the soiled swatches, each swatch is measured once in the center of the soiled area, then the average value is calculated. In the case of the clean swatches, each swatch is measured once on each side, then the average value is calculated. The reflectance measurements are all made at 500nm.

Soiled swatches: Soiled swatches are made using beta-glucan (from barley) and carbon black ("carbon for detergency tests" supplied by Sentaku Kagaku Kyokai, Tokyo, Japan). Dissolve about 0.67g of beta-glucan in 100 mL tap water by stirring and warming to >50°C. Add 0.33g carbon black. Blend with an UltraTurrax® T25 blender, speed 4000 rpm for 2 minutes. Apply 250 microL of the beta-glucan/carbon onto the center of each swatch. Allow to dry overnight at room temperature.

The swatches used in this example had an average reflectance value of 93.5 before soil application and 17.5 after soiling.

Endo-glucanase addition: The endo-glucanase from Example 2 was added to give an activity concentration of 0, 20 or 100 ECU per liter of detergent solution. The activity units, ECU, are determined by method SM-0302, as above.

Results: Detergent without bleach (average of reflectance measurements after washing)

| Endo-glucanase added | Soiled swatches | Clean swatches |
|---|---|---|
| 0 | 25.1 | 33.5 |
| 20 ECU per liter | 35.7 | 46.7 |
| 100 ECU per liter | 40.2 | 59.1 |

Results: Detergent with bleach (average of reflectance measurements after washing)

| Endo-glucanase added | Soiled swatches | Clean swatches |
|---|---|---|
| 0 | 24.6 | 27.7 |
| 20 ECU per liter | 36.8 | 52.6 |
| 100 ECU per liter | 39.3 | 63.2 |

The endo-glucanase increased the removal of soil from the fabric, as seen by the increased reflectance value of the stained swatches after washing with endo-glucanase as compared to the result after washing without endo-glucanase. The endo-glucanase also decreases the soil redeposition, as seen by the increased reflectance value of the clean swatches after washing with endo-glucanase. The improvements of soil removal and anti-redeposition provided by the endo-glucanase are essentially unchanged by the addition of the bleach.

**EXAMPLE 4: Anti-redeposition effect**

Clean cotton fabric is washed together with soiled cotton fabric in a solution of a household detergent. The wash is carried out in a Terg-O-Tometer. During the wash, soil is released from the soiled fabric into the detergent liquor. This soil can then redeposit onto the clean cotton. After washing, the cotton fabrics are rinsed and dried, and then measured with a reflectance spectrophotometer in order to detect the degree of soil redeposition.
Detergent: Powder household detergent, Asian.
Detergent concentration: 0.67g/l in water with hardness 4°dH.
1000 mL of detergent solution per T-O-T beaker.
Cotton fabric: Total of 33g fabric per T-O-T beaker, comprising suitably sized pieces of:
white woven cotton, #2003 (Tanigashira, Osaka, Japan), total weight 11g

white cotton interlock, total weight 13g
soiled cotton fabric, type EMPA101 (EMPA, Switzerland), total weight 9g.
Wash: Temperature 25°C, wash time 40 minutes, at 125 rpm. After washing the #2003 cotton is rinsed under running tap water for 10 minutes, then dried.

Reflectance measurements. The pieces of #2003 woven cotton are measured, on both sides, using a Macbeth® 7000 reflectance spectrophotometer, 500nm. The average result for measurements from each T-O-M beaker is calculated.

Enzyme addition: In this trial, the glucanase prepared as described in Example 2 was added to the detergent liquor before the start of the wash step.

*Results:*

| Endo-glucanase added (ECU per liter) | Reflectance of #2003, at 500nm |
|---|---|
| 0 | 76.67 |
| 0 | 76.05 |
| 1 | 81.86 |
| 5 | 84.30 |
| 20 | 84.85 |
| 50 | 85.99 |

The activity units, ECU, are determined by method SM-0302, as above. From the results it can be concluded that addition of the endo-glucanase reduces the soil redeposition.

**EXAMPLE 5: LAS stability of enzyme samples**

The LAS stability % was determined by the procedure described above. The following enzymes were tested:

Carezyme is the trade name for enzyme products from Novozymes A/S that contain (as the only enzyme component) the 43 kD cellulase derived from *Humicola insolens* DSM 1800 disclosed in WO 91/17243 (SEQ ID NO: 2). Detergent compositions containing this enzyme are disclosed in EP 822 973.
Renozyme is the trade name for enzyme products from Novozymes A/S that contain (as the only enzyme component) the *Thielavia terrestris* cellulase variant disclosed in Example 1 of WO 98/12307.
Endo-glucanase. This is an endo-glucanase with anti-redeposition effect as defined above. A method for preparation of this enzyme is described in Example 2.

Results:

| Enzyme | LAS stability % |
|---|---|
| Carezyme | 13 |
| Renozyme | 62 |
| Endo-glucanase | 83 |

These results show that, according to the definition used, Carezyme is not LAS stable and that both Renozyme and the endo-glucanase are LAS stable.

**EXAMPLE 6: Wash test: Renozyme® and endo-glucanase**

The purpose of this test was to measure the enzyme detergency benefit of Renozyme® alone and of Renozyme® in combination with endo-glucanase.

For this test the soiled swatches were prepared as follows: Swatches of the pre-washed #2003 fabric, prepared as above, were soiled as follows. A suspension of 210 mg carbon black ("carbon for detergency tests", supplied by Sentakukagaku-kyokai, 2-11-1 Shimomaruko Ohta-ku, Tokyo 146-8620, Japan) was prepared in 75 ml tetrachloroethylene (Fluka®, cat. nr. 86972) by strong stirring. The cotton swatches were placed flat on a horizontal metal surface. 300μl of the carbon suspension was pipetted onto the centre of each cotton swatch. The soiled cotton swatches were allowed to dry at room temperature overnight.

For this test the detergent composition was as described above and with the following surfactants and pH:

LAS: 0.5g per liter
Nonionic: none
pH: 9.5

Enzyme detergency benefit results:

| | Renozyme® at 50 μg/l | Renozyme® at 125 μg/l |
|---|---|---|
| No endo-glucanase | 6.1 | 8.1 |
| endo-glucanase at 75μg/l | 15.4 | |
| endo-glucanase at 190 μg/l | | 16.9 |

The enzyme concentrations are specified here in terms of the equivalent concentration of the pure, catalytically active enzyme proteins, in order to avoid ambiguity resulting from activity assay procedures. Standard biochemical techniques can be used to purify and characterise the enzymes.

A suitable cellulase can be obtained from samples of the commercial product Renozyme® from Novozymes A/S or derived as described in Example 1 of WO 98/12307.

The endo-glucanase is an endo-glucanase with anti-redeposition effect as defined above. A method for preparation of this enzyme is described in Example 2.

The results show that the combinations of Renozyme® and the endo-glucanase give a higher enzyme detergency benefit than Renozyme® alone in a detergent solution that contains LAS but no other surfactants.

**EXAMPLE 7: Wash test: Stainzyme® and endo-glucanase**

The purpose of this test was to measure the enzyme detergency benefit of Stainzyme® alone and of Stainzyme® in combination with endo-glucanase on a soil that contains starch.

Stainzyme® is the trade name of a commercially available alpha-amylase product produced by Novozymes A/S. Stainzyme® is intended for use in detergents for laundry and hard surface cleaning.

For this test the soiled swatches were prepared as follows: Swatches of the pre-washed #2003 fabric, prepared as above, were soiled as follows. A suspension of 4.5g of potato starch ("kartoffelmel", produced by KMC kartoffelmelcentralen, DK-7400 Herning, Denmark, starch content approximately 80%) was prepared in 500ml tap water with stirring, then heated to boiling and then cooled. To 100ml of this solution, 332 mg of carbon black ("carbon for detergency tests", supplied by Sentakukagaku-kyokai, 2-11-1 Shimomaruko Ohta-ku, Tokyo 146-8620, Japan) were added, and the suspension was homogenised with an UltraTurrax® blender. The cotton swatches were placed flat on a horizontal metal surface. 250μl of the carbon/starch suspension was pipetted onto the centre of each cotton swatch. The soiled cotton swatches were allowed to dry at room temperature overnight.

For this test the detergent composition was as described above and with the following surfactants and pH:

LAS: 0.4g per liter
Nonionic: 0.2g per liter
pH: 9.5

Enzyme detergency benefit results:

| | Stainzyme® at 2.1 mg/l | Stainzyme® at 5.3 mg/l |
|---|---|---|
| No endo-glucanase | -2.9 | 1.7 |
| endo-glucanase at 75 μg/l | 10.1 | |
| endo-glucanase at 190 μg/l | | 13.1 |

The enzyme concentrations are specified here in terms of the equivalent concentration of the pure, catalytically active enzyme proteins, in order to avoid ambiguity resulting from activity assay procedures. Standard biochemical techniques can be used to purify and characterise the enzymes.

A suitable amylase can be obtained from samples of the commercial product Stainzyme® from Novozymes A/S or derived from *B. licheniformis* variants as disclosed in WO 01/66712 and WO 01/64852.

The endo-glucanase is an endo-glucanase with anti-redeposition effect as defined above. A method for preparation of this enzyme is described in Example 2.

In this test the soil contains starch. The results show that the combinations of endo-glucanase and Stainzyme® give a higher enzyme detergency benefit than Stainzyme® alone.

**EXAMPLE 8: Wash test: Savinase® and endo-glucanase**

The purpose of this test was to measure the enzyme detergency benefit of Savinase alone and of Savinase in combination with endo-glucanase on a soil that contains protein.

Savinase® is the trade name of subtilisin 309. It is a commercially available protease product produced by Novozymes A/S. Savinase® is intended for use in detergents for laundry and hard surface cleaning.

For this test the soiled swatches were prepared as follows: Swatches of the pre-washed #2003 fabric, prepared as above, were soiled as follows. A solution of 7.2g of gelatine (gelatine from porcine skin, Fluka®, cat. number 04055) was prepared in 100ml tap water by warming and with stirring. 10 ml of this solution was diluted to 50ml with water and 165 mg of carbon black ("carbon for detergency tests", supplied by Sentakukagaku-kyokai, 2-11-1 Shimomaruko Ohta-ku, Tokyo 146-8620, Japan) were added, and the suspension was homogenised with an UltraTurrax® blender. The cotton swatches were placed flat on a horizontal metal surface. 250μl of the carbon/gelatine suspension was pipetted onto the centre of each cotton swatch. The soiled cotton swatches were allowed to dry at room temperature overnight.

For this test the detergent composition was as described above and with the following surfactants and pH:

LAS: 0.4g per liter
Nonionic: 0.2g per liter
pH: 9.5

Enzyme detergency benefit results:

| | Savinase® at 0.8 mg/l | Savinase® at 1.9 mg/l |
|---|---|---|
| No endo-glucanase | 3.7 | 7.6 |
| Endo-glucanase at 75 μg/l | 16.6 | |
| Endo-glucanase at 190 μg/l | | 17.7 |

The enzyme concentrations are specified here in terms of the equivalent concentration of the pure, catalytically active enzyme proteins, in order to avoid ambiguity resulting from activity assay procedures. Standard biochemical techniques can be used to purify and characterise the enzymes.

A suitable protease can be obtained from samples of the commercial product Savinase® from Novozymes A/S or derived from *Bacillus lentus* NCIB 10309 as described in US 3,723,250.

The endo-glucanase is an endo-glucanase with anti-redeposition effect as defined above. A method for preparation of this enzyme is described in Example 2.

In this test the soil contains protein. The results show that the combinations of the endo-glucanase and Savinase® give a higher enzyme detergency benefit than Savinase® alone.

**EXAMPLE 9: Wash test: Mannanase and endo-glucanase**

The purpose of this test was to measure the enzyme detergency benefit of a mannanase alone and of a mannanase in combination with endo-glucanase on a soil that contains galactomannan.

For this test the soiled swatches were prepared as follows: Swatches of the pre-washed #2003 fabric, prepared as above, were soiled as follows. A solution of 667 mg of guar gum (Sigma®, G 4129) was prepared in 200ml tap water by warming and with stirring and 332 mg of carbon black ("carbon for detergency tests", supplied by Sentakukagaku-kyokai, 2-11-1 Shimomaruko Ohta-ku, Tokyo 146-8620, Japan) were added, and the suspension was homogenised with an UltraTurrax® blender. The cotton swatches were placed flat on a horizontal metal surface. 500μl of the carbon/guar gum suspension was pipetted onto the centre of each cotton swatch. The soiled cotton swatches were allowed to dry

at room temperature overnight.

For this test the detergent composition was as described above and with the following surfactants and pH:

LAS: 0.4g per liter
Nonionic: 0.2g per liter
pH: 9.5

Enzyme detergency benefit results:

| | Mannanase at 0.3 mg/l | Mannanase at 0.75 mg/l |
|---|---|---|
| No endo-glucanase | 4.8 | 6.2 |
| Endo-glucanase at 75 µg/l | 16.2 | |
| Endo-glucanase at 190 µg/l | | 12.6 |

The enzyme concentrations are specified here in terms of the equivalent concentration of the pure, catalytically active enzyme proteins, in order to avoid ambiguity resulting from activity assay procedures. Standard biochemical techniques can be used to purify and characterise the enzymes.

A suitable mannanase can be obtained from *Bacillus* sp.1633, disclosed in WO 99/64619. Preferred is the mannanase of SEQ ID NO: 2 of WO 99/64619.

The endo-glucanase is an endo-glucanase with anti-redeposition effect as defined above. A method for preparation of this enzyme is described in Example 2.

In this test the soil contains a galactomannan. The results show that the combinations of the endo-glucanase and mannanase give a higher enzyme detergency benefit than mannanase alone.

**EXAMPLE 10: Wash test: Pectate Lyase and endo-glucanase**

The purpose of this test was to measure the enzyme detergency benefit of a pectate lyase (BioPrep® L) alone and in combination with endo-glucanase on a soil that contains pectin.

BioPrep® is the trade name of a commercially available pectate lyase product produced by Novozymes A/S. BioPrep® is intended for use in cleaning of cotton fibres.

For this test the soiled swatches were prepared as follows: Swatches of the pre-washed #2003 fabric, prepared as above, were soiled as follows. A solution of 150 mg of pectin (Sigma®, P 9311) was prepared in 50ml tap water by warming and with stirring and 83 mg of carbon black ("carbon for detergency tests", supplied by Sentakukagaku-kyokai, 2-11-1 Shimomaruko Ohta-ku, Tokyo 146-8620, Japan) were added, and the suspension was homogenised with an UltraTurrax® blender. The cotton swatches were placed flat on a horizontal metal surface. 500µl of the carbon/pectin suspension was pipetted onto the centre of each cotton swatch. The soiled cotton swatches were allowed to dry at room temperature overnight.

For this test the detergent composition was as described above and with the following surfactants and pH:

LAS: 0.4g per liter
Nonionic: 0.2g per liter
pH: 9.5

Enzyme detergency benefit results:

| | BioPrep® at 1 mg/l | BioPrep® at 2.5 mg/l |
|---|---|---|
| No endo-glucanase | -8.0 | -5.1 |
| Endo-glucanase at 75 µg/l | 6.6 | |
| Endo-glucanase at 190 µg/l | | 6.7 |

The enzyme concentrations are specified here in terms of the equivalent concentration of the pure, catalytically active enzyme proteins, in order to avoid ambiguity resulting from activity assay procedures. Standard biochemical techniques can be used to purify and characterise the enzymes.

A suitable pectate lyase can be obtained from *Bacillus licheniformis,* as described in US 6,124,127.

The endo-glucanase is an endo-glucanase with anti-redeposition effect as defined above. A method for preparation of this enzyme is described in Example 2.

In this test the soil contains a pectin. The results show that the combinations of the endo-glucanase and pectate lyase give a higher enzyme detergency benefit than the pectate lyase alone.

## EXAMPLE 11: Wash test: Lipex® and endo-glucanase

The purpose of this test was to measure the enzyme detergency benefit of a lipase (Lipex®) alone and in combination with endo-glucanase on a soil that contains triglyceride oils.

Lipex® is the trade name of a commercially available lipase product produced by Novozymes A/S. Lipex® is intended for use in detergents for laundry and hard surface cleaning.

For this test the soiled swatches were prepared as follows: Swatches of the pre-washed #2003 fabric, prepared as above, were soiled as follows. A suspension of 1.5 g of oat flakes (rolled, toasted oats, containing approximately 7% fats by weight, e.g. from Fællesindkøb I/S, 2605 Brøndby, Denmark) was prepared in 500ml tap water by stirring and heating to boiling and homogenising with an UltraTurrax® blender and then cooling. To 100ml of this suspension 166 mg of carbon black ("carbon for detergency tests", supplied by Sentakukagaku-kyokai, 2-11-1 Shimomaruko Ohta-ku, Tokyo 146-8620, Japan) were added, and the suspension was homogenised with an UltraTurrax blender. 25ml of corn oil (refined corn oil, for household use, 100% oil by weight) were added and the mixture was homogenised with an UltraTurrax® blender. The cotton swatches were placed flat on a horizontal metal surface. 100μl of the carbon/oil suspension was pipetted onto the centre of each cotton swatch. The soiled cotton swatches were allowed to dry at room temperature overnight.

For this test the detergent composition was as described above and with the following surfactants and pH:

LAS: 0.4g per liter
Nonionic: 0.2g per liter
pH: 9.5

Enzyme detergency benefit results:

| | Lipex® at 140 μg/l | Lipex® at 710 μg/l |
|---|---|---|
| No endo-glucanase | -1.7 | -4.0 |
| Endo-glucanase at 75 μg/l | 46.4 | |
| Endo-glucanase at 190 μg/l | | 53.9 |

The enzyme concentrations are specified here in terms of the equivalent concentration of the pure, catalytically active enzyme proteins, in order to avoid ambiguity resulting from activity assay procedures. Standard biochemical techniques can be used to purify and characterise the enzymes.

The lipase can either be obtained from samples of the commercial product Lipex® from Novozymes A/S or derived from suitable *Humicola lanuginosa* variants as described in WO 00/60063.

The endo-glucanase is an endo-glucanase with anti-redeposition effect as defined above. A method for preparation of this enzyme is described in Example 2.

In this test the soil contains a triglyceride oil. The results show that the combinations of the endo-glucanase and Lipex® give a higher enzyme detergency benefit than Lipex® alone.

## EXAMPLE 12: Tests for endo-glucanase with anti-redeposition effect

Two enzymes were evaluated by the tests for "endo-glucanase with anti-redeposition effect". These were:

The enzyme that can be prepared as described in Example 2 above ("Example 2 enzyme"), and
Carezyme, i.e. the 43 kD cellulase derived from *Humicola insolens.*

Results:

| | deltaOD from test for endo-glucanase activity | Anti-redeposition effect |
|---|---|---|
| Example 2 enzyme | 0.4 | 10 |
| Carezyme | >1 | 0 |

The results show that the Example 2 enzyme is an anti-redeposition endo-glucanase, i.e. an endo-glucanase with anti-redeposition effect according to the invention, and that Carezyme is not.

## Claims

1. Use of a detergent composition comprising an anti-redeposition endo-glucanase having the amino acid sequence of SEQ ID NO: 2, or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1 and an alpha-amylase to provide improved detergency performance on stains that contain starch, said use being **characterized in that** the detergency performance obtained from the combination of an anti-redeposition endo-glucanase and an alpha-amylase is higher than the detergency performance of the same detergent

composition without the anti-redeposition endo-glucanase and said detergency performance is determined by the wash test method of Example 7.

2. The use according to claim 1, wherein the alpha-amylase is derived from a strain of the genus *Bacillus.*

3. The use according to claim 2, wherein the alpha-amylase is derived from a strain of the genus *Bacillus,* preferably a strain of *Bacillus* sp., especially *Bacillus* sp: DSM 12649, NCIB 12512, or NCIB 12513.

4. 4 The use according to claims 1 to 3, wherein the endo-glucanase derived from *Bacillus* sp. AA349, DSM 12648 (SEQ ID NO: 2) and the alpha-amylase is an alpha-amylase derived from *Bacillus* sp. DSM 12649.

5. The use according to any of claims 1 to 4, wherein the enzyme detergency benefit from the enzyme combination is at least 5 units higher than the enzyme detergency benefit of the same detergent composition without the anti-redeposition endo-glucanase.

6. The use according to anyone of the claims 1 to 5, wherein the endo-glucanase is active at a pH in the range of 4-11.

7. The use according to anyone of the claims 1 to 6, wherein the composition further comprises one or more enzymes selected from the group consisting of cellulases, beta-glucanases, hemicellulases, lipases, peroxidases, laccases, glucoamylases, cutinases, pectinases, reductases, oxidases, phenoloxidases, ligninases, pullulanases, pectate lyases, xyloglucanases, xylanases, pectin acetyl esterases, polygalacturonases, rhamnogalacturonases, pectin lyases, other mannanases, pectin methylesterases, cellobiohydrolases, transglutaminases, or mixtures thereof.

8. A process for washing a fabric, comprising contacting a fabric with an aqueous solution of a detergent composition comprising an anti-redeposition endo-glucanase having the amino acid sequence of SEQ ID NO: 2; or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1 and an alpha-amylase, for an effective period of time.

9. The process according to claim 8, wherein the period of time is between 2 minutes and 24 hours.

10. The process according to claim 9, wherein the period of time is from 5 to 60 minutes at a temperature in the range of 10° to 60°C.

11. The process according to claim 8 or 9, wherein the weight ratio of the endo-glucanase protein component to the total enzyme protein is less than 1:2.

12. A process for washing a hard surface, comprising contacting the surface with an aqueous solution of a detergent composition comprising an anti-redeposition endo-glucanase having the amino acid sequence of SEQ ID NO: 2; or an endo-glucanase having a sequence of at least 90% identity to the amino acid sequence of position 1 to position 773 of SEQ ID NO:2; or a fragment thereof that has endo-glucanase activity, when identity is determined by GAP provided in the GCG program package using a GAP creation penalty of 3.0 and GAP extension penalty of 0.1 and an alpha-amylasefor an effective period of time.

13. The process according to claim 12, wherein the period of time is between 1 minute and 1 hour.

14. The process according to claim 12 or 3, wherein the weight ratio of the endo-glucanase protein component to the total enzyme protein is less than 1:2.

**Patentansprüche**

1. Verwendung einer Detergenzzusammensetzung, umfassend eine Anti-Redepositions-Endoglucanase mit der Aminosäuresequenz von SEQ ID NO: 2, oder eine Endoglucanase mit einer Sequenz mit mindestens 90% Identität zu der Aminosäuresequenz in Position 1 bis Position 773 von SEQ ID NO: 2; oder ein Fragment davon, das Endoglucanase-Aktivität aufweist, wenn Identität bestimmt wird durch GAP, bereitgestellt in dem GCG-Programmpaket unter Verwendung eines GAP creation penalty von 3,0 und GAP extension penalty von 0,1 und einer Alpha-Amylase

um verbesserte Detergenzleistung auf Flecken, die Stärke enthalten, bereitzustellen, wobei die Verwendung **dadurch gekennzeichnet ist, dass** die Reinigungsleistung die durch die Kombination einer Anti-Redepositions-Endoglucanase und einer Alpha-Amylase erhalten wird, höher ist als die Reinigungsleistung der gleichen Detergenzzusammensetzung ohne die Anti-Redepositions-Endoglucanase und die Reinigungsleistung bestimmt wird durch das Waschtestverfahren von Beispiel 7.

**2.** Verwendung nach Anspruch 1, wobei die Alpha-Amylase von einem *Bacillus* Stamm abgeleitet ist.

**3.** Verwendung nach Anspruch 2, wobei die Alpha-Amylase von einem *Bacillus* Stamm abgeleitet ist, vorzugsweise einem *Bacillus sp.* Stamm, insbesondere *Bacillus sp.* DSM 12649, NCIB 12512, oder NCIB 12513.

**4.** Verwendung nach Ansprüchen 1 bis 3, wobei die Endoglucanase abgeleitet von *Bacillus sp.* AA349, DSM 12648 (SEQ ID NO: 2) und die Alpha-Amylase eine Alpha-Amylase abegeleitet von *Bacillus sp.* DSM 12649 ist.

**5.** Verwendung nach einem beliebigen der Ansprüche 1 bis 4, wobei der Reinigungsvorteil des Enzyms aus der Enzymkombination mindestens 5 Einheiten höher ist als der Reinigungsvorteil des Enzyms der gleichen Detergenzzusammensetzung ohne die Anti-Redepositions-Endoglucanase.

**6.** Verwendung nach einem beliebigen der Ansprüche 1 bis 5, wobei die Endo-glucanase bei einem pH in dem Bereich von 4-11 aktiv ist.

**7.** Verwendung nach einem beliebigen der Ansprüche 1 bis 6, wobei die Zusammensetzung des Weiteren ein oder mehrere Enzym(e) umfasst, ausgewählt aus der Gruppe bestehend aus Cellulasen, Beta-Glucanasen, Hemicellulasen, Lipasen, Peroxidasen, Laccasen, Glucoamylasen, Cutinasen, Pektinasen, Reduktasen, Oxidasen, Phenoloxidasen, Ligninasen, Pullulanasen, Pektatlyasen, Xyloglucanasen, Xylanasen, Pektinacetylesterasen, Polygalacturonasen, Rhamnogalacturonasen, Pektinlyasen, andere Mannanasen, Pektinmethylesterasen, Cellobiohydrolasen, Transglutaminasen oder Mischungen davon.

**8.** Verfahren zum Waschen eines Gewebes, umfassend In-Kontakt-Bringen eines Gewebes mit einer wässrigen Lösung einer Detergenzzusammensetzung, umfassend eine Anti-Redepositions-Endoglucanase mit der Aminosäuresequenz von SEQ ID NO: 2, oder eine Endoglucanase mit einer Sequenz mit mindestens 90% Identität zu der Aminosäuresequenz von Position 1 bis Position 773 von SEQ ID NO: 2; oder ein Fragment davon, das Endoglucanase-Aktivität aufweist, wenn Identität bestimmt wird durch GAP bereitgestellt durch das GCG-Programmpaket unter Verwendung eines GAP creation penalty von 3,0 und GAP extension penalty von 0,1 und einer Alpha-Amylase, für einen effektiven Zeitraum, umfasst.

**9.** Verfahren nach Anspruch 8, wobei der Zeitraum zwischen 2 Minuten und 24 Stunden liegt.

**10.** Verfahren nach Anspruch 9, wobei der Zeitraum von 5 bis 60 Minuten bei einer Temperatur in dem Bereich von 10°C bis 60°C liegt.

**11.** Verfahren nach Anspruch 8 oder 9, wobei das Gewichtsverhältnis der Endoglucanaseprotein-Komponente zu dem gesamten Enzymprotein weniger als 1:2 beträgt.

**12.** Verfahren zum Reinigen einer harten Oberfläche, umfassend In-Kontakt-Bringen der Oberfläche mit einer wässrigen Lösung einer Detergenzzusammensetzung, umfassend eine Anti-Redepositions-Endoglucanase mit der Aminosäuresequenz von SEQ ID NO: 2, oder eine Endoglucanase mit einer Sequenz mit mindestens 90% Identität zu der Aminosäuresequenz von Position 1 bis Position 773 von SEQ ID NO: 2; oder ein Fragment davon, das Endoglucanase-Aktivität aufweist, wenn Identität bestimmt wird durch GAP, bereitgestellt in dem GCG-Programmpaket unter Verwendung eines GAP creation penalty von 3,0 und GAP extension penalty von 0,1 und einer Alpha-Amylase, für einen effektiven Zeitraum, umfasst.

**13.** Verfahren nach Anspruch 12, wobei der Zeitraum zwischen 1 Minute und 1 Stunde liegt.

**14.** Verfahren nach Anspruch 12 oder 13, wobei das Gewichtsverhältnis der Endoglucanaseprotein-Komponente zu dem gesamten Enzymprotein weniger als 1:2 beträgt.

## Revendications

**1.** Utilisation d'une composition détergente comprenant une endoglucanase anti-redéposition ayant une séquence d'acides aminés SEQ ID NO 2; ou une endoglucanase ayant une séquence ayant au moins 90% d'identité avec la séquence d'acides aminés allant de la position 1 à la position 773 de SEQ ID NO 2; ou un fragment de celle-ci qui possède une activité d'endoglucanase, lorsque l'identité est déterminée par GAP fourni dans le pack logiciel GCG en utilisant une pénalité de création de GAP de 3,0 et une pénalité d'extension de GAP de 0,1 et une alpha-amylase pour fournir une performance de détergence améliorée sur des taches qui contiennent un amidon, ladite utilisation étant **caractérisée en ce que** la performance de détergence obtenue à partir de la combinaison de l'endoglucanase anti-redéposition et d'une alpha-amylase est supérieure à la performance de détergence de la même composition détergente sans endoglucanase anti-redéposition et ladite performance de détergence est déterminée par la méthode d'essai de lavage de l'exemple 7.

**2.** Utilisation selon la revendication 1, dans laquelle l'alpha-amylase est dérivée d'une souche de *Bacillus.*

**3.** Utilisation selon la revendication 2, dans laquelle l'alpha-amylase est dérivée d'une souche de *Bacillus,* principalement d'une souche de *Bacillus sp.,* particulièrement *Bacillus sp.* DSM 12649, NCIB 12512, ou NCIB 12513.

**4.** Utilisation selon les revendications 1 à 3, dans laquelle l'endoglucanase est dérivée d'une souche de *Bacillus sp.* AA349, DSM 12648 (SEQ ID NO: 2) et l'alpha-amylase est une alpha-amylse dérivée de *Bacillus sp.* DSM 12649.

**5.** Utilisation selon l'une quelconque des revendications 1 à 4, dans laquelle le bénéfice de détergence enzymatique provenant de la combinaison enzymatique est d'au moins 5 unités supérieur au bénéfice de détergence enzymatique de la même composition détergente sans endoglucanase anti-redéposition.

**6.** Utilisation selon l'une quelconque des revendications 1 à 5, dans laquelle l'endoglucanase est active à un pH dans la plage allant de 4 à 11.

**7.** Utilisation selon l'une quelconque des revendications 1 à 6, dans laquelle la composition comprend en outre une ou plusieurs enzymes choisies dans le groupe constitué par les cellulases, les bêta-glucanases, les hémicellulases, les lipases, les peroxydases, les laccases, les glucoamylases, les cutinases, les pectinases, les réductases, les oxydases, les phénoloxydases, les ligninases, les pullulanases, les pectate-lyases, les xyloglucanases, les xylanases, les pectine-acétylestérases, les polygalacturonases, les rhamnogalacturonases, les pectine-lyases, d'autres mannanases, les pectine-méthylestérases, les cellobiohydrolases, les transglutaminases ou des mélanges de celles-ci.

**8.** Procédé de lavage d'un tissu, ledit procédé comprenant la mise en contact d'un tissu avec une solution aqueuse d'une composition détergente comprenant une endoglucanase anti-redéposition ayant la séquence d'acides aminés SEQ ID NO 2; ou une endoglucanase ayant une séquence ayant au moins 90% d'identité avec la séquence d'acides aminés allant de la position 1 à la position 773 de SEQ ID NO 2; ou un fragment de celle-ci qui possède une activité d'endoglucanase, lorsque l'identité est déterminée par GAP fourni dans le pack logiciel GCG en utilisant une pénalité de création de GAP de 3,0 et une pénalité d'extension de GAP de 0,1, et une alpha-amylase, pendant une période de temps efficace.

**9.** Procédé selon la revendication 8, dans lequel la période de temps est comprise entre 2 minutes et 24 heures.

**10.** Procédé selon la revendication 9, dans lequel la période de temps est comprise de 5 à 60 minutes à une température dans la plage allant de 10°C à 60°C.

**11.** Procédé selon la revendication 8 ou 9, dans lequel le rapport massique du composant de protéine d'endoglucanase sur les protéines enzymatiques totales est inférieur à 1:2.

**12.** Procédé de lavage d'une surface dure, ledit procédé comprenant la mise en contact de la surface avec une solution aqueuse d'une composition détergente comprenant une endoglucanase anti-redéposition ayant la séquence d'acides aminés SEQ ID NO 2; ou une endoglucanase ayant une séquence ayant au moins 90% d'identité avec la séquence d'acides aminés allant de la position 1 à la position 773 de SEQ ID NO 2; ou un fragment de celle-ci qui possède une activité d'endoglucanase, lorsque l'identité est déterminée par GAP fourni dans le pack logiciel GCG en utilisant une pénalité de création de GAP de 3,0 et une pénalité d'extension de GAP de 0,1, et une alpha-amylase,

pendant une période de temps efficace.

**13.** Procédé selon la revendication 12, dans lequel la période de temps est comprise entre 1 minute et 1 heure.

**14.** Procédé selon la revendication 12 ou 13, dans lequel le rapport massique du composant de protéine d'endoglucanase sur les protéines enzymatiques totales est inférieur à 1:2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 822973 A
- WO 0209909 A
- EP 1350843 A
- JP 2000210081 A
- WO 9812307 A
- WO 9629397 A
- GB 1296839 A
- WO 9526397 A
- WO 0060060 A
- WO 8906279 A
- WO 8906270 A
- EP 258068 A
- EP 305216 A
- EP 238023 A
- EP 214761 A
- EP 721981 A
- JP 8000426 W
- JP 8000454 W
- EP 571982 A
- WO 9514783 E
- EP 218272 A
- EP 331376 A
- GB 1372034 A
- JP 64744992 B
- WO 9116422 A
- WO 8809367 A
- WO 9009446 A
- WO 9964619 A
- WO 9927083 A
- WO 9927084 A
- WO 0055309 A
- WO 02092741 A
- US 4565647 A
- GB 1082179 A
- US 3929678 A
- US 4228044 A
- EP 000224 A
- BE 831368
- BE 821369
- BE 821370
- DE 2446686
- DE 2446487
- US 3935257 A
- BE 840623
- GB 1379241 A
- NL 7205873
- GB 1387447 A
- GB 1261829 A
- GB 1398421 A
- GB 1398422 A
- US 3936448 A
- GB 1439000 A
- GB 1425343 A
- GB 1596756 A
- US 4483781 A
- EP 0133354 A
- US 4412934 A
- US 4634551 A
- EP 120591 A
- EP 91870207 A
- US SN08136626 A
- EP 0537381 A
- US 4033718 A
- US 3933672 A
- DE 2646126
- EP 0593841 A
- EP 92201649 A
- GB 1464616 A
- US 3455838 A
- US 4116885 A
- US 4711730 A
- EP 0272033 A
- EP 311342 A
- GB 1400898 A
- US 5019292 A
- GB 514276 A1
- EP 0011340 A
- EP 0026528 B
- EP 0242919 A
- EP 0299575 A
- EP 0313146 A
- WO 0060063 A
- US 4435307 A
- EP 0495257 A
- WO 9412621 A
- WO 9501426 A
- US 4106991 A
- US 4661452 A
- GB 1483591 A
- WO 9701629 A
- WO 9526397 A1
- EP 0506780 A
- WO 9109129 A
- WO 0075344 A1
- WO 9117243 A
- WO 0166712 A
- WO 0164852 A
- US 3723250 A

- US 6124127 A


**Non-patent literature cited in the description**


- **DARTOIS et al.** *Biochemica et Biophysica Acta,* 1993, vol. 1131, 253-260
- *The Journal of the American Oil Chemists Society,* 1975, vol. 52, 323-329
- **SCHWARTZ ; PERRRY ; BERCH.** *Surface Active Agents and Detergents,* vol. I, II
- Efficient manganese catalysts for low-temperature bleaching. *Nature,* 1994, vol. 369, 637-639
- **DARTOIS et al.** *Biochemica et Biophysica acta,* 1993, vol. 1131, 253-260
- **YAMAGUCHI et al.** *Gene,* 1991, vol. 103, 61-67
- **SCHIMADA, Y. et al.** *J. Biochem.,* 1989, vol. 106, 383-388
- **HASS, M.J et al.** *Gene,* 1991, vol. 109, 117-113
- **KUGIMIYA et al.** *Biosci. Biotech. Biochem.,* 1992, vol. 56, 716-719
- **DIDERICHSEN, B. ; WEDSTED, U. ; HEDEGAARD, L. ; JENSEN, B. R. ; SJØHOLM, C.** Cloning of aldB, which encodes alpha-acetolactate decarboxylase, an exoenzyme from Bacillus brevis. *J. Bacteriol.,* 1990, vol. 172, 4315-4321
- Bacillus subtilis and other Gram-Positive Bacteria. American Society for microbiology, 1993, 618
- **YASBIN, R.E. ; WILSON, G.A. ; YOUNG, F.E.** Transformation and transfection in lysogenic strains of Bacillus subtilis: evidence for selective induction of prophage in competent cells. *J. Bacteriol,* 1975, vol. 121, 296-304
- **SAMBROOK et al.** Molecular cloning: A laboratory manual. Cold Spring Harbor lab, 1989
- Current protocols in Molecular Biology. John Wiley and Sons, 1995
- Molecular Biological Methods for Bacillus. John Wiley and Sons, 1990
- **MCKENZIE, T. et al.** *Plasmid,* 1986, vol. 15, 93-103
- **P.L. JØRGENSEN et al.** *Gene,* 1990, vol. 96, 37-41
- **PITCHER, D. G. ; SAUNDERS, N. A. ; OWEN, R. J.** Rapid extraction of bacterial genomic DNA with guanidium thiocyanate. *Lett Appl Microbiol,* 1989, vol. 8, 151-156
- **JAY C. HARRIS.** Detergency Evaluation and Testing. Interscience Publishers Ltd, 1954, 60-61

SEQUENCE LISTING

<110> Novozymes A/S

<120> Detergent composition

<130> 10383

<160> 12

<170> PatentIn version 3.2

<210> 1
<211> 2322
<212> DNA
<213> Bacillus sp.

<220>
<221> CDS
<222> (1)..(2322)

<400> 1

```
gca gaa gga aac act cgt gaa gac aat ttt aaa cat tta tta ggt aat      48
Ala Glu Gly Asn Thr Arg Glu Asp Asn Phe Lys His Leu Leu Gly Asn
1               5                   10                  15

gac aat gtt aaa cgc cct tct gag gct ggc gca tta caa tta caa gaa      96
Asp Asn Val Lys Arg Pro Ser Glu Ala Gly Ala Leu Gln Leu Gln Glu
            20                  25                  30

gtc gat gga caa atg aca tta gta gat caa cat gga gaa aaa att caa      144
Val Asp Gly Gln Met Thr Leu Val Asp Gln His Gly Glu Lys Ile Gln
        35                  40                  45

tta cgt gga atg agt aca cac gga tta caa tgg ttt cct gar atc ttg      192
Leu Arg Gly Met Ser Thr His Gly Leu Gln Trp Phe Pro Glu Ile Leu
    50                  55                  60

aat gat aac gca tac aaa gct ctt gct aac gat tgg gaa tca aat atg      240
Asn Asp Asn Ala Tyr Lys Ala Leu Ala Asn Asp Trp Glu Ser Asn Met
65                  70                  75                  80

att cgt cta gct atg tat gtc ggt gaa aat ggc tat gct tca aat cca      288
Ile Arg Leu Ala Met Tyr Val Gly Glu Asn Gly Tyr Ala Ser Asn Pro
                85                  90                  95

gag tta att aaa agc aga gtc att aaa gga ata gat ctt gct att gaa      336
Glu Leu Ile Lys Ser Arg Val Ile Lys Gly Ile Asp Leu Ala Ile Glu
            100                 105                 110

aat gac atg tat gtt att gtt gat tgg cat gta cat gca cct ggt gat      384
Asn Asp Met Tyr Val Ile Val Asp Trp His Val His Ala Pro Gly Asp
        115                 120                 125

cct aga gat ccc gtt tac gct gga gca gaa gat ttc ttt aga gat att      432
Pro Arg Asp Pro Val Tyr Ala Gly Ala Glu Asp Phe Phe Arg Asp Ile
    130                 135                 140

gca gca tta tat cct aac aat cca cac att att tat gag tta gcg aat      480
Ala Ala Leu Tyr Pro Asn Asn Pro His Ile Ile Tyr Glu Leu Ala Asn
145                 150                 155                 160
```

```
gag cca agt agt aac aat aat ggt gga gct ggg att cca aat aat gaa      528
Glu Pro Ser Ser Asn Asn Asn Gly Gly Ala Gly Ile Pro Asn Asn Glu
                165                 170                 175

gaa ggt tgg aat gcg gta aaa gaa tac gct gat cca att gta gaa atg      576
Glu Gly Trp Asn Ala Val Lys Glu Tyr Ala Asp Pro Ile Val Glu Met
            180                 185                 190

tta cgt gat agc ggg aac gca gat gac aat atc atc att gtg ggt agt      624
Leu Arg Asp Ser Gly Asn Ala Asp Asp Asn Ile Ile Ile Val Gly Ser
        195                 200                 205

cca aac tgg agt cag cgt cct gac tta gca gct gat aat cca att aat      672
Pro Asn Trp Ser Gln Arg Pro Asp Leu Ala Ala Asp Asn Pro Ile Asn
    210                 215                 220

gat cac cat aca atg tat act gtt cac ttc tac act ggt tca cat gct      720
Asp His His Thr Met Tyr Thr Val His Phe Tyr Thr Gly Ser His Ala
225                 230                 235                 240

gct tca act gag agc tat ccg cct gaa act cct aac tct gaa aga gga      768
Ala Ser Thr Glu Ser Tyr Pro Pro Glu Thr Pro Asn Ser Glu Arg Gly
                245                 250                 255

aac gta atg agt aac act cgt tat gcg tta gaa aac gga gta gcg gta      816
Asn Val Met Ser Asn Thr Arg Tyr Ala Leu Glu Asn Gly Val Ala Val
            260                 265                 270

ttt gcg aca gaa tgg gga aca agt caa gca aat gga gat ggt ggt cct      864
Phe Ala Thr Glu Trp Gly Thr Ser Gln Ala Asn Gly Asp Gly Gly Pro
        275                 280                 285

tat ttt gat gaa gca gat gta tgg att gag ttt tta aat gaa aac aac      912
Tyr Phe Asp Glu Ala Asp Val Trp Ile Glu Phe Leu Asn Glu Asn Asn
    290                 295                 300

att agt tgg gct aac tgg tct tta acg aat aaa aat gaa gtg tct ggt      960
Ile Ser Trp Ala Asn Trp Ser Leu Thr Asn Lys Asn Glu Val Ser Gly
305                 310                 315                 320

gca ttt aca cca ttc gag tta ggt aag tct aac gca acc aat ctt gac     1008
Ala Phe Thr Pro Phe Glu Leu Gly Lys Ser Asn Ala Thr Asn Leu Asp
                325                 330                 335

cca ggt cca gat cat gtg tgg gca cca gaa gag tta agt ctt tcg gga     1056
Pro Gly Pro Asp His Val Trp Ala Pro Glu Glu Leu Ser Leu Ser Gly
            340                 345                 350

gaa tat gta cgt gct cgt att aaa ggt gtg aac tat gag cca atc gac     1104
Glu Tyr Val Arg Ala Arg Ile Lys Gly Val Asn Tyr Glu Pro Ile Asp
        355                 360                 365

cgt aca aaa tac acg aaa gta ctt tgg gac ttt aat gat gga acg aag     1152
Arg Thr Lys Tyr Thr Lys Val Leu Trp Asp Phe Asn Asp Gly Thr Lys
    370                 375                 380

caa gga ttt gga gtg aat tcg gat tct cca aat aaa gaa ctt att gca     1200
Gln Gly Phe Gly Val Asn Ser Asp Ser Pro Asn Lys Glu Leu Ile Ala
385                 390                 395                 400

gtt gat aat gaa aac aac act ttg aaa gtt tcg gga tta gat gta agt     1248
Val Asp Asn Glu Asn Asn Thr Leu Lys Val Ser Gly Leu Asp Val Ser
                405                 410                 415
```

```
aac gat gtt tca gat ggc aac ttc tgg gct aat gct cgt ctt tct gcc      1296
Asn Asp Val Ser Asp Gly Asn Phe Trp Ala Asn Ala Arg Leu Ser Ala
                420                 425                 430

gac ggt tgg gga aaa agt gtt gat att tta ggt gct gag aag ctt aca      1344
Asp Gly Trp Gly Lys Ser Val Asp Ile Leu Gly Ala Glu Lys Leu Thr
            435                 440                 445

atg gat gtt att gtt gat gaa cca acg acg gta gct att gcg gcg att      1392
Met Asp Val Ile Val Asp Glu Pro Thr Thr Val Ala Ile Ala Ala Ile
    450                 455                 460

cca caa agt agt aaa agt gga tgg gca aat cca gag cgt gct gtt cga      1440
Pro Gln Ser Ser Lys Ser Gly Trp Ala Asn Pro Glu Arg Ala Val Arg
465                 470                 475                 480

gtg aac gcg gaa gat ttt gtt cag caa acg gac ggt aag tat aaa gct      1488
Val Asn Ala Glu Asp Phe Val Gln Gln Thr Asp Gly Lys Tyr Lys Ala
                485                 490                 495

gga tta aca att aca gga gaa gat gct cct aac cta aaa aat atc gct      1536
Gly Leu Thr Ile Thr Gly Glu Asp Ala Pro Asn Leu Lys Asn Ile Ala
            500                 505                 510

ttt cat gaa gaa gat aac aat atg aac aac atc att ctg ttc gtg gga      1584
Phe His Glu Glu Asp Asn Asn Met Asn Asn Ile Ile Leu Phe Val Gly
        515                 520                 525

act gat gca gct gac gtt att tac tta gat aac att aaa gta att gga      1632
Thr Asp Ala Ala Asp Val Ile Tyr Leu Asp Asn Ile Lys Val Ile Gly
    530                 535                 540

aca gaa gtt gaa att cca gtt gtt cat gat cca aaa gga gaa gct gtt      1680
Thr Glu Val Glu Ile Pro Val Val His Asp Pro Lys Gly Glu Ala Val
545                 550                 555                 560

ctt cct tct gtt ttt gaa gac ggt aca cgt caa ggt tgg gac tgg gct      1728
Leu Pro Ser Val Phe Glu Asp Gly Thr Arg Gln Gly Trp Asp Trp Ala
                565                 570                 575

gga gag tct ggt gtg aaa aca gct tta aca att gaa gaa gca aac ggt      1776
Gly Glu Ser Gly Val Lys Thr Ala Leu Thr Ile Glu Glu Ala Asn Gly
            580                 585                 590

tct aac gcg tta tca tgg gaa ttt gga tat cca gaa gta aaa cct agt      1824
Ser Asn Ala Leu Ser Trp Glu Phe Gly Tyr Pro Glu Val Lys Pro Ser
        595                 600                 605

gat aac tgg gca aca gct cca cgt tta gat ttc tgg aaa tct gac ttg      1872
Asp Asn Trp Ala Thr Ala Pro Arg Leu Asp Phe Trp Lys Ser Asp Leu
    610                 615                 620

gtt cgc ggt gag aat gat tat gta gct ttt gat ttc tat cta gat cca      1920
Val Arg Gly Glu Asn Asp Tyr Val Ala Phe Asp Phe Tyr Leu Asp Pro
625                 630                 635                 640

gtt cgt gca aca gaa ggc gca atg aat atc aat tta gta ttc cag cca      1968
Val Arg Ala Thr Glu Gly Ala Met Asn Ile Asn Leu Val Phe Gln Pro
                645                 650                 655

cct act aac ggg tat tgg gta caa gca cca aaa acg tat acg att aac      2016
Pro Thr Asn Gly Tyr Trp Val Gln Ala Pro Lys Thr Tyr Thr Ile Asn
```

```
                660                 665                 670

ttt gat gaa tta gag gaa gcg aat caa gta aat ggt tta tat cac tat      2064
Phe Asp Glu Leu Glu Glu Ala Asn Gln Val Asn Gly Leu Tyr His Tyr
        675                 680                 685

gaa gtg aaa att aac gta aga gat att aca aac att caa gat gac acg      2112
Glu Val Lys Ile Asn Val Arg Asp Ile Thr Asn Ile Gln Asp Asp Thr
    690                 695                 700

tta cta cgt aac atg atg atc att ttt gca gat gta gaa agt gac ttt      2160
Leu Leu Arg Asn Met Met Ile Ile Phe Ala Asp Val Glu Ser Asp Phe
705                 710                 715                 720

gca ggg aga gtc ttt gta gat aat gtt cgt ttt gag ggg gct gct act      2208
Ala Gly Arg Val Phe Val Asp Asn Val Arg Phe Glu Gly Ala Ala Thr
                725                 730                 735

act gag ccg gtt gaa cca gag cca gtt gat cct ggc gaa gag acg cca      2256
Thr Glu Pro Val Glu Pro Glu Pro Val Asp Pro Gly Glu Glu Thr Pro
            740                 745                 750

cct gtc gat gag aag gaa gcg aaa aaa gaa caa aaa gaa gca gag aaa      2304
Pro Val Asp Glu Lys Glu Ala Lys Lys Glu Gln Lys Glu Ala Glu Lys
        755                 760                 765

gaa gag aaa gaa gag taa                                              2322
Glu Glu Lys Glu Glu
    770
```

<210> 2
<211> 773
<212> PRT
<213> Bacillus sp.

<400> 2

```
Ala Glu Gly Asn Thr Arg Glu Asp Asn Phe Lys His Leu Leu Gly Asn
1               5                   10                  15

Asp Asn Val Lys Arg Pro Ser Glu Ala Gly Ala Leu Gln Leu Gln Glu
            20                  25                  30

Val Asp Gly Gln Met Thr Leu Val Asp Gln His Gly Glu Lys Ile Gln
        35                  40                  45

Leu Arg Gly Met Ser Thr His Gly Leu Gln Trp Phe Pro Glu Ile Leu
    50                  55                  60

Asn Asp Asn Ala Tyr Lys Ala Leu Ala Asn Asp Trp Glu Ser Asn Met
65                  70                  75                  80

Ile Arg Leu Ala Met Tyr Val Gly Glu Asn Gly Tyr Ala Ser Asn Pro
                85                  90                  95

Glu Leu Ile Lys Ser Arg Val Ile Lys Gly Ile Asp Leu Ala Ile Glu
```

100 105 110

Asn Asp Met Tyr Val Ile Val Asp Trp His Val His Ala Pro Gly Asp
115 120 125

Pro Arg Asp Pro Val Tyr Ala Gly Ala Glu Asp Phe Phe Arg Asp Ile
130 135 140

Ala Ala Leu Tyr Pro Asn Asn Pro His Ile Ile Tyr Glu Leu Ala Asn
145 150 155 160

Glu Pro Ser Ser Asn Asn Asn Gly Gly Ala Gly Ile Pro Asn Asn Glu
165 170 175

Glu Gly Trp Asn Ala Val Lys Glu Tyr Ala Asp Pro Ile Val Glu Met
180 185 190

Leu Arg Asp Ser Gly Asn Ala Asp Asp Asn Ile Ile Ile Val Gly Ser
195 200 205

Pro Asn Trp Ser Gln Arg Pro Asp Leu Ala Ala Asp Asn Pro Ile Asn
210 215 220

Asp His His Thr Met Tyr Thr Val His Phe Tyr Thr Gly Ser His Ala
225 230 235 240

Ala Ser Thr Glu Ser Tyr Pro Pro Glu Thr Pro Asn Ser Glu Arg Gly
245 250 255

Asn Val Met Ser Asn Thr Arg Tyr Ala Leu Glu Asn Gly Val Ala Val
260 265 270

Phe Ala Thr Glu Trp Gly Thr Ser Gln Ala Asn Gly Asp Gly Gly Pro
275 280 285

Tyr Phe Asp Glu Ala Asp Val Trp Ile Glu Phe Leu Asn Glu Asn Asn
290 295 300

Ile Ser Trp Ala Asn Trp Ser Leu Thr Asn Lys Asn Glu Val Ser Gly
305 310 315 320

Ala Phe Thr Pro Phe Glu Leu Gly Lys Ser Asn Ala Thr Asn Leu Asp
325 330 335

Pro Gly Pro Asp His Val Trp Ala Pro Glu Glu Leu Ser Leu Ser Gly
340 345 350

Glu Tyr Val Arg Ala Arg Ile Lys Gly Val Asn Tyr Glu Pro Ile Asp
355 360 365

Arg Thr Lys Tyr Thr Lys Val Leu Trp Asp Phe Asn Asp Gly Thr Lys
370 375 380

Gln Gly Phe Gly Val Asn Ser Asp Ser Pro Asn Lys Glu Leu Ile Ala
385 390 395 400

Val Asp Asn Glu Asn Asn Thr Leu Lys Val Ser Gly Leu Asp Val Ser
405 410 415

Asn Asp Val Ser Asp Gly Asn Phe Trp Ala Asn Ala Arg Leu Ser Ala
420 425 430

Asp Gly Trp Gly Lys Ser Val Asp Ile Leu Gly Ala Glu Lys Leu Thr
435 440 445

Met Asp Val Ile Val Asp Glu Pro Thr Thr Val Ala Ile Ala Ala Ile
450 455 460

Pro Gln Ser Ser Lys Ser Gly Trp Ala Asn Pro Glu Arg Ala Val Arg
465 470 475 480

Val Asn Ala Glu Asp Phe Val Gln Gln Thr Asp Gly Lys Tyr Lys Ala
485 490 495

Gly Leu Thr Ile Thr Gly Glu Asp Ala Pro Asn Leu Lys Asn Ile Ala
500 505 510

Phe His Glu Glu Asp Asn Asn Met Asn Asn Ile Ile Leu Phe Val Gly
515 520 525

Thr Asp Ala Ala Asp Val Ile Tyr Leu Asp Asn Ile Lys Val Ile Gly
530 535 540

Thr Glu Val Glu Ile Pro Val Val His Asp Pro Lys Gly Glu Ala Val
545 550 555 560

Leu Pro Ser Val Phe Glu Asp Gly Thr Arg Gln Gly Trp Asp Trp Ala
565 570 575

Gly Glu Ser Gly Val Lys Thr Ala Leu Thr Ile Glu Glu Ala Asn Gly
580 585 590

Ser Asn Ala Leu Ser Trp Glu Phe Gly Tyr Pro Glu Val Lys Pro Ser
595 600 605

```
Asp Asn Trp Ala Thr Ala Pro Arg Leu Asp Phe Trp Lys Ser Asp Leu
    610                 615                 620

Val Arg Gly Glu Asn Asp Tyr Val Ala Phe Asp Phe Tyr Leu Asp Pro
625                 630                 635                 640

Val Arg Ala Thr Glu Gly Ala Met Asn Ile Asn Leu Val Phe Gln Pro
                645                 650                 655

Pro Thr Asn Gly Tyr Trp Val Gln Ala Pro Lys Thr Tyr Thr Ile Asn
            660                 665                 670

Phe Asp Glu Leu Glu Glu Ala Asn Gln Val Asn Gly Leu Tyr His Tyr
        675                 680                 685

Glu Val Lys Ile Asn Val Arg Asp Ile Thr Asn Ile Gln Asp Asp Thr
    690                 695                 700

Leu Leu Arg Asn Met Met Ile Ile Phe Ala Asp Val Glu Ser Asp Phe
705                 710                 715                 720

Ala Gly Arg Val Phe Val Asp Asn Val Arg Phe Glu Gly Ala Ala Thr
                725                 730                 735

Thr Glu Pro Val Glu Pro Glu Pro Val Asp Pro Gly Glu Glu Thr Pro
            740                 745                 750

Pro Val Asp Glu Lys Glu Ala Lys Lys Glu Gln Lys Glu Ala Glu Lys
        755                 760                 765

Glu Glu Lys Glu Glu
    770
```

<210> 3
<211> 1174
<212> DNA
<213> Thielavia terrestris

<220>
<221> CDS
<222> (60)..(956)

<400> 3

```
gagcagcacc cctcaagctg tacagtttcc accccgctct cttttcttcg gcccccagg      59

atg cgc tct act ccc gtt ctt cgc aca acc ctg gcc gct gca ctt cct      107
Met Arg Ser Thr Pro Val Leu Arg Thr Thr Leu Ala Ala Ala Leu Pro
1               5                   10                  15

ctg gtc gcc tcc gcg gcc agt ggc agt ggc cag tcc acg aga tac tgg      155
```

```
Leu Val Ala Ser Ala Ala Ser Gly Ser Gly Gln Ser Thr Arg Tyr Trp
            20                  25                  30

gac tgc tgc aag ccg tcg tgc gct tgg ccc ggg aag gcc gcc gtc agc      203
Asp Cys Cys Lys Pro Ser Cys Ala Trp Pro Gly Lys Ala Ala Val Ser
        35                  40                  45

caa ccg gtc tac gcg tgc gat gcc aac ttc cag cgc ctg tcc gac ttc      251
Gln Pro Val Tyr Ala Cys Asp Ala Asn Phe Gln Arg Leu Ser Asp Phe
    50                  55                  60

aat gtc cag tcg ggc tgc aac ggc ggc tcg gcc tac tcc tgc gcc gac      299
Asn Val Gln Ser Gly Cys Asn Gly Gly Ser Ala Tyr Ser Cys Ala Asp
65                  70                  75                  80

cag act ccc tgg gcg gtg aac gac aat ctc gcc tac ggc ttc gcc gcg      347
Gln Thr Pro Trp Ala Val Asn Asp Asn Leu Ala Tyr Gly Phe Ala Ala
                85                  90                  95

acg agc atc gcc ggc ggg tcc gaa tcc tcg tgg tgc tgc gcc tgc tac      395
Thr Ser Ile Ala Gly Gly Ser Glu Ser Ser Trp Cys Cys Ala Cys Tyr
            100                 105                 110

gcg ctc acc ttc act tcc ggt ccc gtc gcc ggc aag aca atg gtg gtg      443
Ala Leu Thr Phe Thr Ser Gly Pro Val Ala Gly Lys Thr Met Val Val
        115                 120                 125

cag tca acg agc act ggc ggc gac ctg gga agt aac cag ttc gat atc      491
Gln Ser Thr Ser Thr Gly Gly Asp Leu Gly Ser Asn Gln Phe Asp Ile
    130                 135                 140

gcc atg ccc ggc ggc ggc gtg ggc atc ttc aac ggc tgc agc tcg cag      539
Ala Met Pro Gly Gly Gly Val Gly Ile Phe Asn Gly Cys Ser Ser Gln
145                 150                 155                 160

ttc ggc ggc ctc ccc ggc gct caa tac ggc ggc att tcg tcg cgc gac      587
Phe Gly Gly Leu Pro Gly Ala Gln Tyr Gly Gly Ile Ser Ser Arg Asp
                165                 170                 175

cag tgc gat tcc ttc ccc gcg ccg ctc aag ccc ggc tgc cag tgg cgg      635
Gln Cys Asp Ser Phe Pro Ala Pro Leu Lys Pro Gly Cys Gln Trp Arg
            180                 185                 190

ttt gac tgg ttc cag aac gcc gac aac ccg acg ttc acg ttc cag cag      683
Phe Asp Trp Phe Gln Asn Ala Asp Asn Pro Thr Phe Thr Phe Gln Gln
        195                 200                 205

gtg cag tgc ccc gcc gag atc gtt gcc cgc tcc ggc tgc aag cgc aac      731
Val Gln Cys Pro Ala Glu Ile Val Ala Arg Ser Gly Cys Lys Arg Asn
    210                 215                 220

gac gac tcc agc ttc ccc gtc ttc acc ccc cca agc ggt ggc aac ggt      779
Asp Asp Ser Ser Phe Pro Val Phe Thr Pro Pro Ser Gly Gly Asn Gly
225                 230                 235                 240

ggc acc ggg acg ccc acg tcg act gcg cct ggg tcg ggc cag acg tct      827
Gly Thr Gly Thr Pro Thr Ser Thr Ala Pro Gly Ser Gly Gln Thr Ser
                245                 250                 255

ccc ggc ggc ggc agt ggc tgc acg tct cag aag tgg gct cag tgc ggt      875
Pro Gly Gly Gly Ser Gly Cys Thr Ser Gln Lys Trp Ala Gln Cys Gly
            260                 265                 270
```

```
ggc atc ggc ttc agc gga tgc acc acc tgt gtc tct ggc acc acc tgc      923
Gly Ile Gly Phe Ser Gly Cys Thr Thr Cys Val Ser Gly Thr Thr Cys
    275                 280                 285

cag aag ttg aac gac tac tac tcg cag tgc ctc taaacagctt ttcgcacgag    976
Gln Lys Leu Asn Asp Tyr Tyr Ser Gln Cys Leu
290                 295

gtggcgggac ggagcaagga gaccgtcaac ttcgtcatgc atatttttg agcgctcaat     1036

acatacataa ccttcgattc ttgtacatag cacgccggta cacatctcac accgactttg    1096

ggggcggaat caggcccgtt ttaaaaaaa aaaaaaaaa aaaaaaaaa aaaaaaaaa        1156

aaaaaaaaa aaaaaaa                                                     1174
```

<210> 4
<211> 299
<212> PRT
<213> Thielavia terrestris

<400> 4

```
Met Arg Ser Thr Pro Val Leu Arg Thr Thr Leu Ala Ala Ala Leu Pro
1               5                   10                  15

Leu Val Ala Ser Ala Ala Ser Gly Ser Gly Gln Ser Thr Arg Tyr Trp
            20                  25                  30

Asp Cys Cys Lys Pro Ser Cys Ala Trp Pro Gly Lys Ala Ala Val Ser
        35                  40                  45

Gln Pro Val Tyr Ala Cys Asp Ala Asn Phe Gln Arg Leu Ser Asp Phe
    50                  55                  60

Asn Val Gln Ser Gly Cys Asn Gly Gly Ser Ala Tyr Ser Cys Ala Asp
65                  70                  75                  80

Gln Thr Pro Trp Ala Val Asn Asp Asn Leu Ala Tyr Gly Phe Ala Ala
                85                  90                  95

Thr Ser Ile Ala Gly Gly Ser Glu Ser Ser Trp Cys Cys Ala Cys Tyr
            100                 105                 110

Ala Leu Thr Phe Thr Ser Gly Pro Val Ala Gly Lys Thr Met Val Val
        115                 120                 125

Gln Ser Thr Ser Thr Gly Gly Asp Leu Gly Ser Asn Gln Phe Asp Ile
    130                 135                 140

Ala Met Pro Gly Gly Gly Val Gly Ile Phe Asn Gly Cys Ser Ser Gln
145                 150                 155                 160
```

```
Phe Gly Gly Leu Pro Gly Ala Gln Tyr Gly Gly Ile Ser Ser Arg Asp
                165                 170                 175

Gln Cys Asp Ser Phe Pro Ala Pro Leu Lys Pro Gly Cys Gln Trp Arg
            180                 185                 190

Phe Asp Trp Phe Gln Asn Ala Asp Asn Pro Thr Phe Thr Phe Gln Gln
        195                 200                 205

Val Gln Cys Pro Ala Glu Ile Val Ala Arg Ser Gly Cys Lys Arg Asn
    210                 215                 220

Asp Asp Ser Ser Phe Pro Val Phe Thr Pro Pro Ser Gly Gly Asn Gly
225                 230                 235                 240

Gly Thr Gly Thr Pro Thr Ser Thr Ala Pro Gly Ser Gly Gln Thr Ser
                245                 250                 255

Pro Gly Gly Gly Ser Gly Cys Thr Ser Gln Lys Trp Ala Gln Cys Gly
            260                 265                 270

Gly Ile Gly Phe Ser Gly Cys Thr Thr Cys Val Ser Gly Thr Thr Cys
        275                 280                 285

Gln Lys Leu Asn Asp Tyr Tyr Ser Gln Cys Leu
    290                 295
```

<210> 5
<211> 42
<212> DNA
<213> Artificial

<220>
<223> Primer

<220>
<221> misc_feature
<222> (1)..(42)
<223> PRIMER LWN5494

<400> S
gtcgccgggg cggccgctat caattggtaa ctgtatctca gc 42

<210> 6
<211> 64
<212> DNA
<213> Artificial

<220>
<223> Primer

<220>
<221> mist_feature
<222> (1)..(64)

<223> Primer LWN5495

<400> 6

gtcgcccggg agctctgatc aggtaccaag cttgtcgacc tgcagaatga ggcagcaaga 60

agat 64

<210> 7
<211> 61
<212> DNA
<213> artificial

<220>
<223> Primer

<220>
<221> misc_feature
<222> (1)..(61)
<223> PRIMER LWN5938

<400> 7

gtcggcggcc gctgatcacg taccaagctt gtcgacctgc agaatgaggc agcaagaaga 60

t 61

<210> 8
<211> 35
<212> DNA
<213> artificial

<220>
<223> Primer

<220>
<221> misc_feature
<222> (1)..(35)
<223> PRIMER LWN5939

<400> 8
gtcggagctc tatcaattgg taactgtatc tcagc 35

<210> 9
<211> 35
<212> DNA
<213> Artificial

<220>
<223> Primer

<220>
<221> misc_feature
<222> (1)..(35)
<223> PRIMER LWN7864

<400> 9
aacagctgat cacgactgat cttttagctt ggcac 35

<210> 10
<211> 37
<212> DNA
<213> Artificial

<220>
<223> Primer

<220>
<221> misc_feature
<222> (1)..(37)
<223> PRIMER LWN7901

<400> 10
aactgcagcc gcggcacatc ataatgggac aaatggg 37

<210> 11
<211> 42
<212> DNA
<213> Artificial

<220>
<223> Primer

<220>
<221> misc_feature
<222> (1)..(42)
<223> Primer 168684

<400> 11
cattctgcag ccgcggcagc agaaggaaac actcgtgaag ac 42

<210> 12
<211> 44
<212> DNA
<213> Artificial

<220>
<223> Primer

<220>
<221> misc_feature
<222> (1)..(44)
<223> Primer 168685

<400> 12
gcgttgagac gcgcggccgc ttactcttct ttctcttctt tctc 44